# EUROPÄISCHE PATENTSCHRIFT

(11) **EP 0 736 516 B1**
(45) Veröffentlichungstag und Bekanntmachung des Hinweises auf die Patenterteilung: **03.02.1999**
(21) Anmeldenummer: 96104868.3
(22) Anmeldetag: 27.03.1996
(51) Int. Cl.: C07C 209/86

(54) **Fraktionierung und Reinigung von aromatischen Polyamingemischen und deren Verwendung**
Fractionation and purification of mixtures of aromatic polyamines and their use
Fractionnement et purification de mélanges de polyamines aromatiques et leur utilisation

(30) Priorität: 07.04.1995 DE 19513069
(43) Veröffentlichungstag der Anmeldung: 09.10.1996
(73) Patentinhaber: BAYER AG, 51368 Leverkusen (DE)
(72) Erfinder: Knöfel, Hartmut, Dr., 51519 Odenthal (DE); Brockelt, Michael, 51379 Leverkusen (DE)

(56) Entgegenhaltungen:
- EP-A- 0 031 423
- EP-A- 0 161 600
- EP-A- 0 288 892
- DE-A- 1 568 087
- DE-A- 2 528 694

## Beschreibung

Die Erfindung betrifft ein Verfahren zur Fraktionierung und Reinigung von aromatischen Polyamingemischen und deren Verwendung.

Die Herstellung von aromatischen Polyaminen und Polyamingemischen, insbesondere der Diphenylmethanreihe wird in zahlreichen Patentmeldungen und Patenten beschrieben, ebenso die Verwendung dieser Produkte. Herausragende Bedeutung kommt dabei der Verwendung dieser Produkte als Rohstoffe für die Herstellung von Isocyanaten zu, in der Regel durch Umsetzung der Polyamingemische mit Phosgen nach dem allgemein üblichen und bekannten Methoden.

Die dabei resultierenden Isocyanate bzw. Isocyanatgemische fallen in vielen Fällen aber nicht in der Form und Zusammensetzung an, wie sie auf der Isocyanatstufe bevorzugt weiterverwendet werden, sondern müssen zuvor durch teilweise aufwendige Aufarbeitungs und Trennverfahren in die verwendungsgerechte Form übergeführt werden. Geeignete Polyaminvorstufen, die weniger aufwendig in die Isocyanatverwendungsformen gebracht werden können, sind in vielen Fällen verfahrenstechnisch schwierig oder nicht zugänglich oder wirtschaftlich unattraktiv herzustellen.

Beispielhaft ist die Gewinnung des für die Herstellung hochwertiger Polyurethanwerkstoffe wichtigen 4,4'-Diisocyanato-diphenylmethans, dessen Aminvorstufe in der Regel aus Anilin und Formaldehyd nur gemeinsam mit Isomeren, insbesondere dem 2,4'-Isomeren, und höherfunktionellen Polyaminen gewonnen werden kann.

Diese Bestandteile sind zwar die Grundlage für die ebenfalls begehrten Isocyanate, jedoch ist die Auftrennung der Rohisocyanate in die für die Weiterverwendung geeigneten Isocyanate bzw. Isocyanatgemische nicht einfach.

In der Regel werden zunächst ein Teil der Zweikernverbindungen von dem Rest abgetrennt. Anschließend wird aus der Zweikernfraktion in einem viele Trennstufen erfordernden zweiten Destillationsschritt das 4,4'-Diisocyanato-diphenylmethan von den anderen Isomeren befreit.

Das 2,4'-Isomere in angereicherter Form hat selbst in neuerer Zeit zunehmende Bedeutung als Polyurethanrohstoff erlangt, kann aber nur mit beträchtlichem destillativen Aufwand gegenüber dem 4,4'-Isomeren angereichert und von dem gegebenenfalls vorhandenen 2,2'-Isomeren befreit werden.

Isomerentrennverfahren oder Anreicherungsverfahren innerhalb der Fraktion der höherkernigen Homologen bzw. der höherfunktionellen Bestandteile der Amine wie auch der Isocyanate der Diphenylmethanreihe sind praktisch nicht bekannt.

Zunehmendes Interesse findet auch das 4,4'-Diamino-diphenylmethan als Rohstoff für das Di-(4-isocyanatocyclohexyl)-methan, die kernhydrierte Form des 4,4'-Diisocyanato-diphenylmethans, wobei die Bereitstellung geeigneter aromatischer Polyamingemische für die Hydrierstufe mit einem möglichst hohen Gehalt an 4,4'-Diamino-diphenylmethan bei gleichzeitig einem möglichst geringen Anteil an 2,4'-Diamino-diphenylmethan sehr aufwendig ist.

Es ist bekannt, daß Amine durch partielle Überführung in ihre Salze in bestimmten Fällen getrennt werden können, wobei u.a. die unterschiedlichen Basenstärken genutzt werden. Dabei handelt es sich in der Regel um Monoamine mit stark unterschiedlichen Basenstärken.

Auch für aromatische Polyamingemische, insbesondere der Diphenylmethanreihe, sind solche Disproportionierungseffekte in zweiphasigen Systemen bereits beschrieben (DE-A 2 238 319 und DE-A 2 528 694).

Die Effekte sind infolge der in einem solchen Gemisch vorhandenen zahlreichen Komponenten, deren Aminogruppen sich vom Typ her - praktisch alle sind Arylaminogruppen - kaum unterscheiden, nicht besonders groß und ausgeprägt, um für eine direkte Nutzung mit einfachen Mitteln interessant zu sein.

Aufgabe war es, ein Verfahren zur Verfügung zu stellen, das es gestattet, aromatische Polyamingemische in einfacher Weise zu fraktionieren bzw. zu reinigen, so daß Isomere in reiner Form oder in angereicherter Form anfallen.

Diese Aufgabe konnte durch das erfindungsgemäße Verfahren, welches aufgrund der erfindungsgemäßen Ausführung überraschend hohe Trennleistung bei der Fraktionierung von aromatischen Polyamingemischen, insbesondere der Diphenylmethanreihe, erzielt, und dabei in der Wirkung weit über die bekannten Effekte des Standes der Technik hinausgeht, gelöst werden.

Bei der erfindungsgemäßen Fraktionierung von aromatischen Polyamingemischen werden andere, unterschiedlich zusammengesetzte Polyamingemische gewonnen.

Bei diesen abgeleiteten Polyamingemischen kann es sich um solche handeln, die auf bekannten Sythesewegen nur sehr aufwendig zugänglich sind. Dabei kann es sich auch um Polyamingemische handeln, die für eine vereinfachte Herstellung der Isocyanate besser geeignet sind, als die bekannten und tehnisch gut herzustellenden Polyamingemische, indem sie z.B. auf der Isocyanatstufe schwierig durchzuführende Isomerentrennung auf der Aminstufe vorwegnehmen. Solche Gemische können auch völlig neuartige, weil nach dem Stand der Technik nicht darstellbare Polyamingemische sein, die zu völlig neuartigen Isocyanaten führen.

Andererseits kann das erfindungsgemäße Verfahren dazu genutzt werden, aus beliebigen, d.h. auch aus wiedergewonnenen Polyamingemischen, die sich durch Verunreinigung oder durch nichtstatistische, d.h. selektive Verluste bei einzelnen Komponenten von den ursprünglich eingesetzten Polyaminen oder Isocyanaten unterscheiden, Produktfraktionen zu gewinnen, welche dem Standard oder den Ausgangspolyaminen entsprechen. Schließlich kann das erfindungsgemäße Verfahren dazu genutzt werden, synthesebedingte und im Endprodukt unerwünschte Neben- und Zwischenprodukte, mitzufraktionieren und in einer Produktfraktion ab und in einer anderen entsprechend anzureichern, gegebenenfalls in einer eigenen Fraktion auszuschleusen.

Gegenstand der Erfindung ist ein Verfahren zur Fraktionierung und Reinigung von aromatischen Polyamingemischen, insbesondere von Polyamingemischen der Diphenylmethanreihe, welches dadurch gekennzeichnet ist, daß man
a) das Polyaminausgangsgemisch (A) in einem zweiphasigen System bestehend aus (i) einer hydrophobem Lösungsmittelphase (B) die im wesentlichen aus hydrophoben Lösungsmittel und gegebenenfalls einem aromatischen Hilfsamin, welches in Wasser praktisch unlöslich ist und unter Normaldruck einen mindestens 20°C unter dem Siedepunkt der am niedrigsten siedenden Komponente des Ausgangsgemisches und mindestens 20°C oberhalb des Siedepunktes des Lösungsmittels liegenden Siedepunkt aufweist, und gegebenenfalls aus Polyamin besteht, und (ii) einer wäßrigen Phase (C), bestehend im wesentlichen aus Wasser, einer starken Säure und gegebenenfalls zumindest teilweise in der Salzform vorliegendem Hilfsamin, sowie gegebenenfalls zumindest teilweise in der Salzform vorliegenden Polyaminen, unter Zuhilfenahme einer nach dem Gegenstromprinzip arbeitenden Extraktionsstufe (7) unter Durchmischung der Phasen verteilt, indem man das Ausgangspolyamingemisch vorzugsweise über die wäßrige Phase in die Extraktionsstufe (7) einbringt mit der Maßgabe, daß in diesem zweiphasigen System die Summe der in den Mengenströmen (A), (B) und (C) eingebrachten Aminäquivalente die Anzahl der im Mengenstrom (C) eingebrachten Säureäquivalente stets übersteigt, die diese Extraktionsstufe (7) verlassende organische Phase (D)
b) gegebenenfalls zumindest teilweise über eine zwischengeschaltete Extraktionsstufe (6) und/oder
c) gegebenenfalls unter Abtrennung eines Teilstromes vor oder nach der gegebenenfalls durchlaufenen Extraktionsstufe (6) und Rückführung des abgetrennten Teilstromes zur Extraktionsstufe (7) und/oder zur gegebenenfalls vorhandenen Extraktionsstufe (6) über eine vorgeschaltete Extraktionsstufe (5),
d) in einer gegebenenfalls mehrstufigen Destillation (11.1),(11.2) in eine erste, in der Extraktionsstufe (7) wiederverwendete Fraktion (E), bestehend im wesentlichen aus hydrophobem Lösungsmittel und gegebenenfalls Hilfsamin, gegebenenfalls eine zweite Fraktion (F), bestehend im wesentlichen aus Hilfsamin und gegebenenfalls hydrophobem Lösungsmittel und einem Destillationsrückstand (G), bestehend im wesentlichen aus einer ersten Polyaminfraktion, auftrennt und
e) die die erste Extraktionsstufe (7) verlassende wäßrige Phase (H) in eine Neutralisationsstufe (8) leitet, mit Basen, vorzugsweise wäßriger Natronlauge, die in der wäßrigen Phase enthaltene Säure neutralisiert und anschließend in einem Phasentrennschritt in eine wäßrige Phase, enthaltend die Säure in Form ihrer neutralen Salze und eine organische Phase, enthaltend im wesentlichen Polyamin, gegebenenfalls Hilfsamin und gegebenenfalls hydrophobes Lösungsmittel, mechanisch auftrennt und
f) die in der Neutralisationsstufe (8) anfallende organische Phase (J) gegebenenfalls nach Durchlaufen einer Waschstufe (9), zumindest teilweise in einer Destillationsstufe (12) aufarbeitet in eine Destillationsfraktion (K), enthaltend die in (J) bzw. (J') enthaltenen Anteile an hydrophobem Lösungsmittel und das gegebenenfalls enthaltene Hilfsamin, und in eine als Destillationsrückstand (L) anfallende zweite Polyaminfraktion.

Das erfindungsgemäße Verfahren wird besonders bevorzugt so durchgeführt, daß
b) die in der Extraktionsstufe (7) anfallende organische Phase(D) zumindest teilweise in einer zwischengeschalteten Extraktionsstufe (6) mit zumindest einer Teilmenge der wäßrigen Säure (Mengenstrom X) und/oder gegebenenfalls Wasser aus Mengenstrom (Y) und/oder gegebenenfalls Hilfsamin im Gegenstrom extrahiert und/oder mit zumindest einer Teilmenge, vorzugsweise mit der Gesamtmenge der in der gegebenenfalls vorhandenen vorgeschalteten Extraktionsstufe (5) anfallenden wäßrigen Phase (Q) im Gegenstrom extrahiert, die in der zwischengeschalteten Extraktionsstufe (6) resultierende wäßrige Phase (N) der Extraktionsstufe (7) zuführt und die in der zwischengeschalteten Extraktionsstufe (6) anfallende organische Phase (O) der Aufarbeitungsstufe (11) zuführt.

Eine weitere verbesserte und daher bevorzugte Ausführungsform des erfindungsgemäßen Verfahrens besteht darin, daß man
c) einen Teilstrom der die Extraktionsstufe (7) verlassenden organischen Phase (D) und/oder
   einen Teilstrom der die gegebenenfalls vorhandene zwischengeschaltete Extraktionsstufe (6) verlassenden organischen Phase (O) abtrennt und in einer vorgeschalteten Extraktionsstufe (5) mit zumindest einer Teilmenge, der als Mengenstrom (X) zur Verfügung stehenden wäßrigen Säure im Gegenstrom extrahiert,
   den in der Extraktionsstufe (5) eingesetzten organischen Mengenstrom (P) so bemißt, daß in (5) ein möglichst weitgehender Übergang des im besagtem organischen Mengenstrom enthaltenen Polyamins in die wäßrige Phase (Q) erfolgt,
   die in der vorgeschalteten Extraktionsstufe (5) resultierende wäßrige Phase (Q) gegebenenfalls nach Zugabe von Wasser aus Mengenstrom (Y) und/oder Hilfsamin der Extraktionsstufe (6) zuführt und
   die in der vorgeschalteten Extraktionsstufe (5) anfallende an Polyamin verarmte organische Phase (R) der Extraktionsstufe (7) und/oder der gegebenenfalls vorhandenen Extraktionsstufe (6) zuführt.

Als Hilfsamine werden vorzugsweise Anilin und/oder am aromatischen Kern alkylsubstituierte Aniline eingesetzt und als Polyamingemisch der Diphenylmethanreihe bevorzugt ein Polyamingemisch, wie es bei der säurekatalysierten Anilin-/Formaldehydkondensation anfällt.

Die derart behandelten Polyamingemische, also die mit dem erfindungsgemäßen Verfahren erzeugten Fraktionen werden zur Herstellung der entsprechenden aromatischen Polyisocyanatgemische und zur Herstellung von Polyurethankunststoffen verwendet.

Außerdem können die nach dem erfindungsgemäßen Verfahren erzeugten Fraktionen zur Herstellung der entsprechenden kernhydrierten Polyamine oder als Vernetzer und als Epoxidhärter verwendet werden.

Die aus den fraktionierten Polyamingemischen hergestellten entsprechenden Polyisocyanate werden bevorzugt zur Herstellung von PU Schaumstoffen eingesetzt.

Ausgangsgemische sind beispielsweise technische Arylamingemische, wie sie bei der Herstellung aus den Ausgangsverbindungen oder wie sie bei der Wiedergewinnung anfallen.

Beispiele von Ausgangsarylamingemischen, für deren Fraktionierung und Reinigung das erfindungsgemäße Verfahren besonders geeignet ist, sind
1. Polyamingemische der Diphenylmethanreihe, wie sie bei der Kondensation und säurekatalysierten Umlagerung von Anilin mit Formaldehyd entstehen
2. Polyamingemische der Diphenylmethanreihe, wie sie bei der säurekatalysierten Kondensation von substituierten Anilinen mit Formaldehyd anfallen,
3. Polyamingemische der Diphenylmethanreihe, wie sie bei der Mischkondensation von substituierten Anilinen untereinander und/oder mit Anilin mit Formaldehyd anfallen,
4. Polyamingemische der Diphenylmethanreihe, wie sie bei der Kondensation, auch der Mischkondensation von substituierten Anilinen und/oder Anilin mit Aldehyden und/oder Ketonen anfallen,
5. Polyamingemische der Diphenylmethanreihe, wie sie bei der Nitrierung und anschließenden Reduktion von Di- und/oder Polyarylmethanen und/oder substituierten Di- und/oder Polyarylmethanen entstehen; unter Polyarylmethanen werden hier insbesondere die Benzylhomologen des Diphenylmethans verstanden,
6. Polyamingemische der Diphenylmethanreihe, wie sie bei der Kondensation von Monoarylmonoaminen (z.B. Anilin, substituierte Aniline) und/oder Monoaryldiaminen (Phenylendiamine, substituierte Phenylendiamine) mit Aldehyden, Ketonen, insbesondere Formaldehyd, und säurekatalysierter Umlagerung entstehen und
7. Polyamingemische der Triphenylmethanreihe, wie sie z.B. bei der Nitrierung und anschließenden Reduktion von Triphenylmethanen und seinen höherkernigen, insbesondere Benzylhomologen entstehen.

Bei den zum Einsatz gelangenden hydrophoben Lösungsmitteln handelt es sich um inerte Lösungsmittel des Siedepunktbereiches von 30 - 280°C, vorzugsweise von 80 - 200°C, wie beispielsweise Chlorbenzol, Dichlorbenzol, Benzol, Toluol, Ethylbenzol, Cumol, Xylol, Dichlorethan, Chloroform und Tetrachlorkohlenstoff. Vorzugsweise werden Xylole, d.h. technische Xylolgemische, insbesondere o-Xylol, Toluol, Ethylbenzol, Cumol und Chlorbenzol eingesetzt.

Bevorzugt werden solche Lösungsmittel verwendet, die ein gutes Lösungsvermögen für die eingesetzten Polyamingemische aufweisen.

Bei den eingesetzten Säuren handelt es sich um wasserlösliche Protonsäuren mit einem unter 2,5 , vorzugsweise unter 1,5 liegenden pKA Wert. Beispiele hierfür sind Salzsäure, Bromwasserstoffsäure, Schwefelsäure, Trifluoressigsäure, Methansulfonsäure oder Phosphorsäure. Bevorzugt eingesetzt werden Salzsäure und Schwefelsäure.

Die genannten Säuren können auch im Gemisch mit sauren oder neutralen Salzen derartiger Säuren, wie z.B. den entsprechenden Ammoniumsalzen oder auch den entsprechenden Alkalisalzen eingesetzt werden.

Im allgemeinen liegen die genannten Säuren in der wäßrigen Phase (C) vor entweder als wäßrige Lösung der freien Säure oder als wäßrige Lösung, die neben der freien Säure auch die Ammoniumsalze der Säure mit Hilfsamin und/oder Polyamin enthält oder als wäßrige Lösung, in der die Säure vollständig in der Form ihrer Ammoniumsalze mit Hilfsamin und/oder Polyamin vorliegt und die gegebenenfalls noch weiteres, nicht salzartig gebundenes Hilfsamin und/oder Polyamin enthält.

Spätestens nach Durchlaufen der Extraktionsstufe (7) liegen die genannten Säuren in der wäßrigen Phase in der Form der Ammoniumsalze der Säure mit der in der wäßrigen Phase befindlichen Fraktion des Polyamins und gegebenenfalls mit Hilfsamin vor.

Nach Durchlaufen der Extraktionsstufe, gegebenenfalls der Extraktionsstufen, wird die in der wäßrigen Phase befindliche Säure durch Neutralisation mit starken Basen in die entsprechenden Neutralsalze übergeführt. Dabei werden die salzartig gebundenen Polyamine und gegebenenfalls Hilfsamin freigesetzt.

Als Hilfsamin finden in der Regel Verwendung Monoarylamine oder Gemische von Monoarylaminen wie z.B. Anilin und/oder am Kern und/oder am Stickstoff alkylsubstituierte Anilinderivate.

Bevorzugt werden primäre Aniline, besonders bevorzugt sind neben Anilin das 2,6-Dimethylanilin, Xylidingemische und 2-Methyl-6-ethylanilin.

Das erfindungsgemäße Verfahren kann sowohl diskontinuierlich als auch kontinuierlich durchgeführt werden. Bevorzugte Ausführungsform ist die kontinuierliche Arbeitsweise. Dabei wird das Verfahren in allen Stufen unter dem Eigendruck des Systems und vorzugsweise in einer Inertgasatmosphäre (Stickstoff) durchgeführt.

Das erfindungsgemäße Verfahren kann sowohl mit einer (Abb. 1) als auch mit zwei (Abb. 2 und Abb. 3) oder drei (Abb. 4) Extraktionsstufen durchgeführt werden.

Das erfindungsgemäße Verfahren kann zur Steigerung des Anreicherungs- bzw. korrespondierenden Abreicherungseffektes mit jeder der anfallenden Produktfraktionen wiederholt werden.

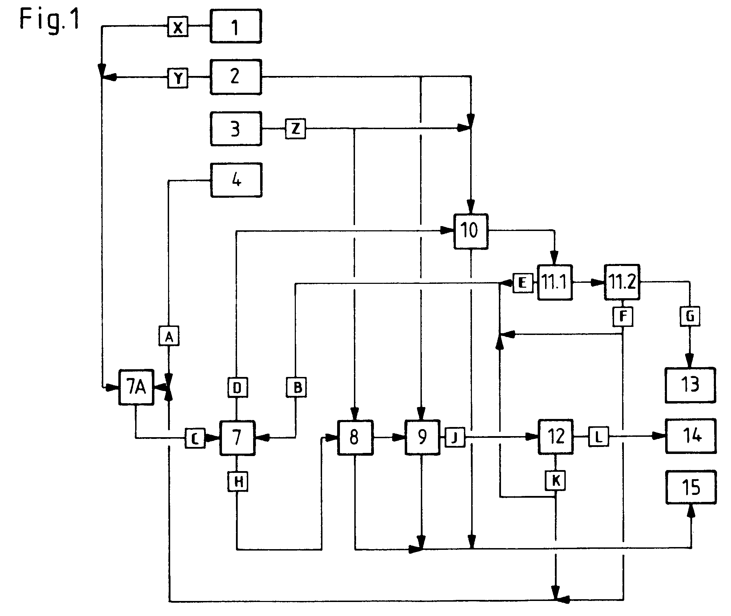

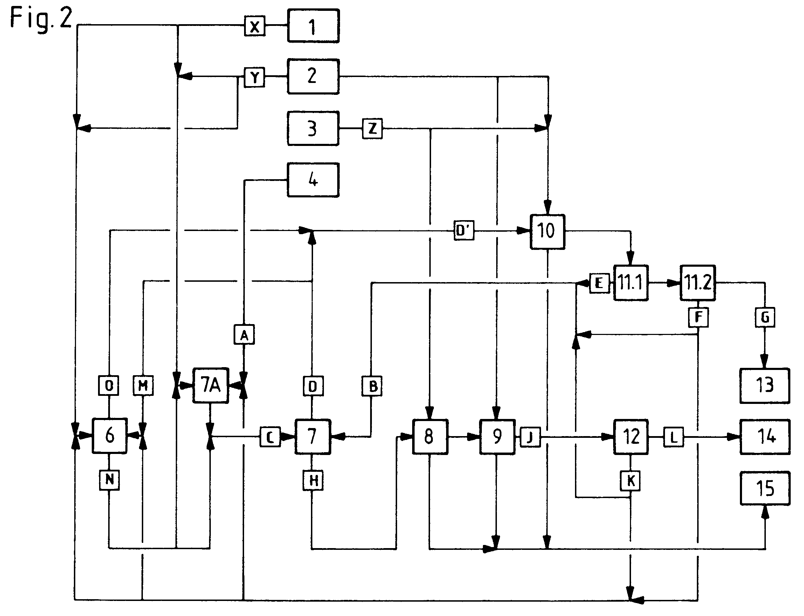

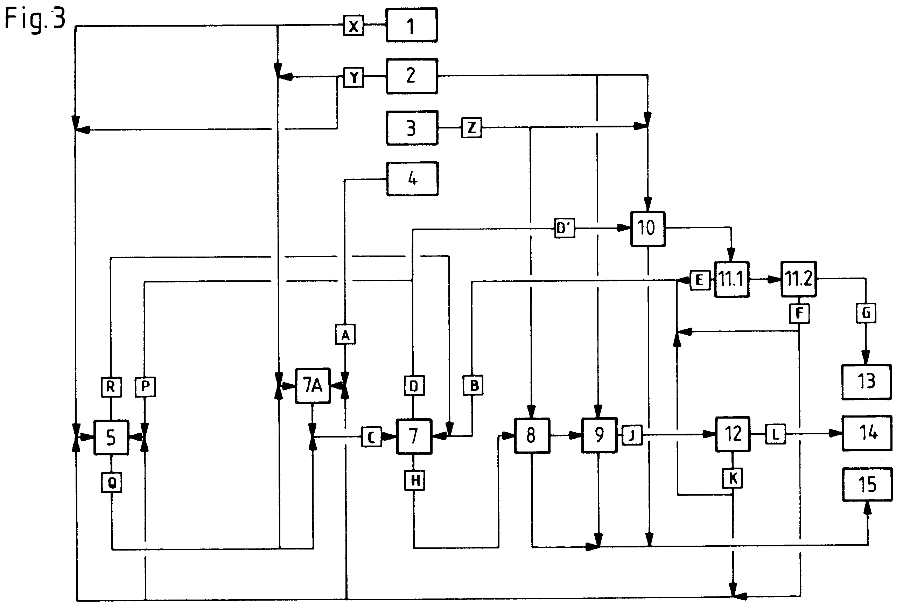

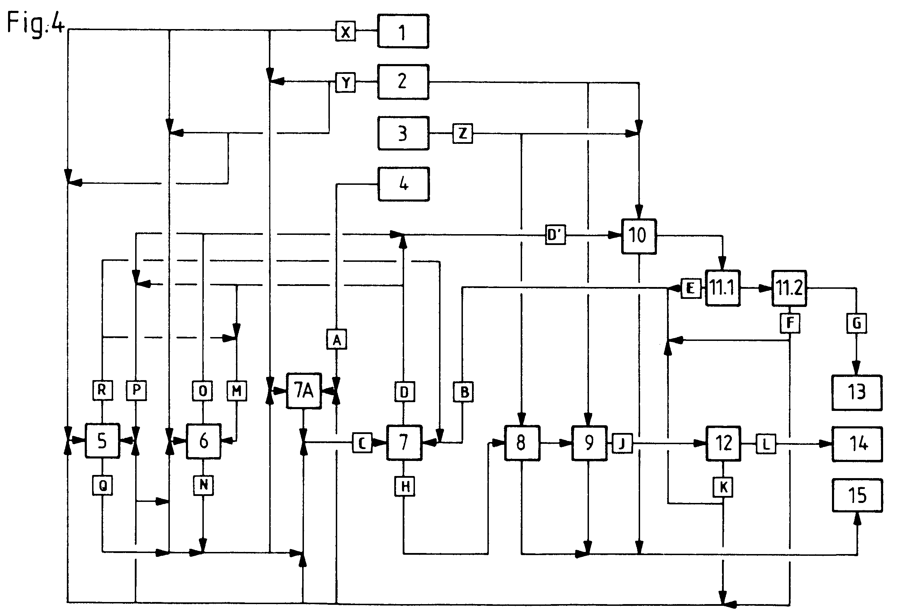

Die in Abb. 1 - 4 dargestellten Fließdiagramme dienen der weiteren Erläuterung des erfindungsgemäßen Verfahrens. In diesen Abbildungen bedeuten :
(1) einen Tank für wäßrige Säure
(2) einen Tank Wasser
(3) einen Tank für wäßrige Base
(4) einen Tank für Ausgangspolyamin
(5) einen ein- oder mehrstufigen Extraktor, dessen aus der Sicht der wäßrigen Phase erste Stufe in der Regel aus einer Mischer Scheidereinheit besteht
(6) eine Extraktionsstufe
(7A) einen Mischer oder eine Mischer Scheidereinheit
(7) eine Extraktionsstufe
(8) eine Neutralisationsstufe
(9) eine Waschstufe
(10) eine Waschstufe
(11.1) eine erste Destillationsstufe einer gegebenenfalls Mehrstufendestillation
(11.2) eine letzte Destillationsstufe einer gegebenenfalls Mehrstufendestillation
(12) eine Destillationsstufe
(13) einen Tank für ein erstes Verfahrensprodukt
(14) einen Tank für ein weiteres Verfahrensprodukt
(15) einen Tank für Abwasser

Die Bezugszeichen A - R und X - Z bezeichnen die Mengenströme, auf die nachstehend und in den Beispielen Bezug genommen wird.

Bei der vorgeschalteten Extraktionsstufe (5) handelt es sich im einfachsten Fall um eine einstufig wirkende Mischer Scheidereinheit, vorzugsweise kommen jedoch mehrstufig wirkende Extraktionseinheiten zum Einsatz, wobei die aus Sicht des Mengenstromes (X) erste Stufe in der Regel aus einer Mischer- Scheidereinheit besteht.

Bei der Extraktionsstufe (6) handelt es sich im einfachsten Fall um eine einstufig wirkende Mischer- Scheidereinheit, vorzugsweise kommen jedoch mehrstufig wirkende Extraktionseinheiten zum Einsatz.

Bei der Stufe (7A) handelt es sich im einfachsten Fall um einen Mischer oder eine Mischer-Scheidereinheit, die gegebenenfalls dem nachfolgenden Extraktor (7) vorgelagert ist.

Die Extraktionsstufe (7) besteht im einfachsten Fall ebenfalls aus einer Mischer-Scheidereinheit, vorzugsweise kommen jedoch auch hier mehrstufig wirkende Extraktionseinheiten zum Einsatz.

Die mehrstufig wirkenden Extraktionseinheiten können aus mehreren in Serie geschalteten Extraktoren bestehen. Vorzugsweise werden die üblichen Gegenstromextraktionsvorrichtungen verwendet.

Bei der Neutralisationsstufe (8) handelt es sich um eine Vorrichtung zur intensiven Durchmischung der wäßrigen Phase (H) zur Umsetzung der enthaltenen Säure mit der wäßrigen Lösung einer starken Base (2) im Überschuß aus Behälter (3) mit der Möglichkeit Neutralisationswärme abzuführen und anschließender Abtrennung des Polyamins.

Zum Durchmischen werden im einfachsten Falle ein oder mehrere gerührte Kessel eingesetzt, dabei kann der Mischvorgang durch Mischdüsen, Intensivmischer und/oder Umpumpvorrichtungen verstärkt werden. Für die anschließende Phasentrennung werden im einfachsten Falle Scheider eingesetzt, wobei die Phasentrennung durch den Einbau von Scheidehilfen verstärkt werden kann. Ebenso geeignet sind beispielsweise Zentrifugen.

In den Fällen, in denen nach der Umsetzung der wäßrigen Phase (H) mit starken Basen die einfache mechanische Abtrennung schwierig oder nicht möglich ist, wird die Abtrennung durch den Einsatz von zusätzlichem hydrophoben Lösungsmittel und/oder Hilfsamin durchgeführt, gegebenenfalls als Extraktionsvorgang in einem vorzugsweise mehrstufig wirkenden Extraktor.

Bei der Waschstufe (9), handelt es sich im einfachsten Fall um eine Mischer Scheidereinheit, in welcher der Mengenstrom (J) mit Wasser gewaschen wird, für die Durchführung des erfindungsgemäßen Verfahrens ist die Waschstufe (9) grundsätzlich nicht erforderlich, in der Regel aber vorteilhaft.

Auch bei der Waschstufe (10) handelt es sich im einfachsten Fall um eine Mischer-Scheidereinheit, vorzugsweise kommen jedoch mehrstufig wirkende Extraktoren zum Einsatz.

Die Waschstufe (10) kann sowohl mit Wasser als auch vorzugsweise mit verdünnten wäßrigen Lösungen starker Basen durchgeführt werden.

Die Destillationsstufe (11) besteht im einfachsten Fall aus einer Destillationskolonne, mit welcher das Zulaufprodukt in eine Destillatfraktion (E), enthaltend das hydrophobe Lösungsmittel und gegebenenfalls vorhandenes Hilfsamin, und den Destillationsrückstand (G), bestehend aus einer ersten Polyaminfraktion, aufgetrennt wird.

Bei der Mitverwendung von Hilfsamin besteht die Destillationsstufe (11) vorzugsweise aus einer wenigstens zweistufigen Mehrstufendestillation, deren erste Stufe (11.1) ein gegenüber dem Zulaufprodukt von Polyamin befreites und an Hilfsamin verarmtes hydrophobes Lösungsmittel als Destillat (E) liefert, und deren letzte Stufe (11.2) ein von Polyamin befreites und an Lösungsmittel verarmtes Hilfsamin als Destillat (F) liefert.

Zusätzlich fällt bei der letzten Destillationsstufe (11.2) die in Mengenstrom (D) enthaltene erste Fraktion des Ausgangspolyamin (A) als Destillationsrückstand (G) an.

Bei der Mitverwendung von Hilfsamin ist die vollständige destillative Auftrennung von hydrophobem Lösungsmittel und Hilfsamin bei Durchführung des erfindungsgemäßen Verfahrens nicht erforderlich.

Die Destillationsstufe (12) besteht im einfachsten Fall aus einer Destillationskolonne. Bei der Durchführung des erfindungsgemäßen Verfahrens ohne Mitverwendung eines Hilfsamins enthält das Zulaufprodukt (J) im einfachsten Fall nur geringe Mengen an hydrophobem Lösungsmittel. Bei Zugabe von hydrophobem Lösungsmittel und/oder Hilfsamin vor oder während der Neutralisationsstufe müssen diese jedoch in (12) wieder abgetrennt werden.

Bei Durchführung der Neutralisationsstufe (8) unter Mitverwendung von zusätzlichem Hilfsamin enthält das Zulaufprodukt zur Destillationsstufe (12) in der Regel beträchtliche Anteile an hydrophobem Lösungsmittel, die im allgemeinen gemeinsam als Destillatfraktion (K) von der als Destillationsrückstand anfallenden zweiten Polyaminfraktion (L) abgetrennt werden.

Erfordert die Durchführung der Neutralisationsstufe (8), auch bei der Mitverwendung von Hilfsamin, den Einsatz von hydrophobem Lösungsmittel in (8) und/oder (9), beispielweise zur Verbesserung der Neutralisationsreaktion oder zur Erleichterung der anschließenden Phasentrennung, dann wird auch die Destillationsstufe (12) vorzugsweise wenigstens zweistufig ausgeführt zur Gewinnung eines weitgehend an hydrophobem Lösungsmittel verarmten Destillates (K) in der letzten hier nicht gezeichneten Stufe (12.2).

Auch aus Gründen einer besseren Energienutzung werden die Destillationsstufen (11) und (12) vorzugsweise mehrstufig durchgeführt.

Das erfindungsgemäße Verfahren kann sowohl unter Verwendung von hydrophobem Lösungsmittel allein als auch unter Verwendung von hydrophobem Lösungsmittel und Hilfsamin durchgeführt werden.

Das erfindungsgemäße Verfahren kann in mehreren technischen Varianten durchgeführt werden.

Gemäß einer ersten Variante erfolgt die Einspeisung des Ausgangspolyamingemisches (Mengenstrom A) aus Behälter (4) in die Verfahrensstufe (7) durch Vermischen mit dem wäßrigen Mengenstrom (C) gegebenenfalls über eine vorgeschaltete Stufe (7A).

Die gegebenenfalls vorhandene Stufe (7A) dient der Entlastung der Extraktionsstufe (7) und besteht im einfachsten Fall der ersten Variante des erfindungsgemäßen Verfahrens aus einem der Stufe (7) vorgeschalteten Mischer, in dem aus wäßriger Säure (Mengenstrom X), gegebenenfalls Wasser (Mengenstrom Y) zur Einstellung einer gewünschten Säurekonzentration und/oder gegebenenfalls Hilfsamin der eigentliche Mengenstrom (C) gebildet und eingestellt wird.

Es hat sich als vorteilhaft erwiesen, auch das Ausgangspolyamin (A) zumindest teilweise bereits im Mischer (7A) mit den Bestandteilen des Mengenstrom (C) zu vermischen und die in diesem Falle resultierende wäßrige Phase als erweiterten Mengenstrom (C) der Extraktionsstufe (7) zuzuführen, bestehend in der Regel aus Wasser, einer starken Säure, Polyamin und gegebenenfalls Hilfsamin.

Im allgemeinen liegt die Säure in der wäßrigen Phase (C) vor als wäßrige Lösung der Säure, die gegebenenfalls Ammoniumsalze der Säure mit Hilfsamin und/oder Polyamin enthält; vorzugsweise liegt die Säure vor als wäßrige Lösung ihrer Ammoniumsalze mit Hilfsamin und/oder Polyamin, die gegebenenfalls freies, d.h. nicht salzartig gebundenes Hilfsamin und/oder Polyamin gelöst enthält.

Es hat sich als zweckmäßig erwiesen, den Säuregehalt der wäßrigen Phase unabhängig von dem sich je nach Verfahrensparametern (z.B. Zusammensetzung von organischer und wäßriger Phase, Phasenverhältnis, Temperatur) in der wäßrigen Phase eines zweiphasigen Systems einstellenden Amingehalt über eine sogenannte "Molarität" zu definieren.

Die "Molarität" wird festgelegt als theoretische Konzentration von zu 100 % protoniertem Amin (d.h.gleiche Anzahl von Säure und Aminäquivalenten) in einem rechnerisch um den Anteil an nicht protonierten Amin vermindertem Volumen oder gegebenenfalls in einem rechnerisch um eine entsprechende Aminmenge bis zur vollständigen Bindung der Säure als Ammoniumsalze erweitertem Volumen an wäßriger Phase.

Die so definierte Molarität kann Werte bis 6 annehmen und wird je nach der jeweiligen Ausführungsform zugrunde liegenden - hier produktbezogenen - Trennaufgabe in diesem Bereich gezielt variiert.

Der für die jeweilige Ausführungsform des erfindungsgemäßen Verfahrens wohldefinierte und in engen Grenzen gemessene und geregelte Säuregehalt des Mengenstromes (C) wird als wichtige Führungsgröße je nach der der jeweiligen Ausführungsform zugrunde liegenden - hier produktbezogen - Trennaufgabe in einem weiten Bereich insgesamt oder in einzelnen Verfahrensstufen gezielt variiert, gegebenenfalls unter Zufuhr von Wasser aus Mengenstrom (Y) oder wäßriger Säure aus Mengenstrom (X).

Nach oben wird dieser Arbeitsbereich praktisch begrenzt einerseits durch zunehmende Kristallisationsneigung der Aminsalze mit zunehmender Konzentration, insbesondere bei hohen Protonierungsgraden, und andererseits durch die zunehmende gegenseitige Löslichkeit der Phasen ineinander, insbesondere bei niedrigen Protonierungsgraden.

Der Protonierungsgrad gibt das Verhältnis von Säureäquivalenten zu Aminäquivalenten wieder.

Nach unten wird dieser Bereich wirtschaftlich begrenzt durch den abnehmenden Säuregehalt und damit die quantitative Abnahme der Trennieistung, d.h. bei hervorragender Trennieistung und technisch problemlos, ist mit sinkender Molarität ein zunehmend größeres Volumen an wäßriger Phase erforderlich für die Trennung einer gegebenen Aminmenge.

In der vorzugsweise mehrstufig betriebenen Extraktionsstufe (7) werden die organische Phase (B) und die wäßrige Phase (C) unter inniger Durchmischung einander entgegengeführt.

Bei diesem Vorgang findet in der Regel ein Übergang von Polyarylamin von der wäßrigen Phase (C) in die organische Phase (B) statt, gegebenenfalls im Austausch gegen Arylamin in entgegengesetzter Richtung.

Voraussetzung für das Zustandekommen des Übergangs von Polyarylamin aus der eingebrachten wäßrigen Phase in die organische Phase ist, daß die Summe der in den Mengenströmen (A), (B)und (C) eingebrachten Aminäquivalente die Anzahl der im Mengenstrom (C) eingebrachten Säureäquivalente stets übersteigt.

Unter dieser Voraussetzung kann die organische Phase (B) im Extremfall allein aus hydrophobem Lösungsmittel bestehen und zu einem guten Trennergebnis führen. In diesem Fall muß in der in die Stufe (7) eingebrachten wäßrigen Phase ein Überschuß der Aminäquivalente über die Säureäquivalente vorliegen.

Enthält die organische Phase (B) neben hydrophobem Lösungsmittel genügend Anteile an Hilfsamin und/oder Polyamin, können auch mit einer in Stufe (7) eingebrachten wäßrigen Phase gute Trennergebnisse erzielt werden, in der die Aminäquivalente im Unterschuß gegenüber den Säureäquivalenten vorliegen.

Selbstverständlich kann auch mit einem Überschuß der Amin- über die Säureäquivalente in der eingebrachten wäßrigen Phase und gleichzeitig einer organischen Phase (B), die neben hydrophobem Lösungsmittel einen Gehalt an Hilfsamin und/oder Polyamin aufweist, ein erwünschtes Trennergebnis erzielt werden.

Darüber hinaus ist es möglich die Verfahrensstufe (7) und das erfindungsgemäße Verfahren insgesamt ohne Verwendung von Hilfsamin durchzuführen.

In diesem Falle ist es besonders vorteilhaft und daher bevorzugt, der organischen Phase (B) als gegebenenfalls enthaltenes Polyamin das als zweite Produktfraktion (L) abgetrennte Polyamin zuzusetzen.

Die organische Phase (B) besteht im allgemeinen aus hydrophobem Lösungsmittel und gegebenenfalls aus Hilfsamin und/oder Polyamin, letzteres vorzugsweise mit der Zusammensetzung des zweiten Verfahrensteilproduktes (L).

Bei der Durchführung des erfindungsgemäßen Verfahrens ohne Verwendung von Hilfsamin wird der Mengenstrom (B) im einfachsten Fall gebildet aus dem Destillatstrom (E), der in diesem Fall gegebenenfalls einstufigen Destillationsstufe (11). Der Mengenstrom (E) besteht in diesem Fall praktisch aus hydrophobem Lösungsmittel. Die Wirksamkeit der Verfahrensstufe (7) ist in diesem Falle an einen genügend großen Überschuß der Aminäquivalente über die Säureäquivalente in der eingebrachten wäßrigen Phase geknüpft, d.h. der Protonierungsgrad in der mit Mengenstrom (A) beaufschlagten, erweiterten wäßrigen Phase (C) muß weniger als 100% betragen.

Diese Forderung ist nicht mehr bindend, wenn die der Stufe (7) zugeführte organische Phase (B) selbst Polyamin enthält, d.h. wenn mit den Phasen (C) und (B) zusammen mehr Aminäquivalente in die Stufe (7) eingeführt werden als Säureäquivalente.

Der organischen Phase (B) Polyamin in Form von Ausgangspolyamin zuzusetzen erhöht zwar den Durchsatz, bringt aber in der Regel in qualitativer Hinsicht ein Trennergebnis, welches bei Mehrstufigkeit der Stufe (7) weitgehend durch das sich einstellende Verteilungsgleichgewicht zwischen den Polyaminkomponenten in der zugeführten organischen Phase (B) und der die Stufe (7) verlassenden wäßrigen Phase (H) kontrolliert und limitiert wird.

In Bezug auf das qualitative Trennergebnis wesentlich effektiver und daher als Ausführungsform bevorzugt ist es, bei Abwesenheit von Hilfsamin eine organische Phase (B) einzusetzen, die das mit der wäßrigen Phase (H) abgetrennte, als zweite Produktfraktion (L) anfallende Polyamin enthält.

Die organische Phase (B) wird in diesem Falle gebildet durch Zugabe einer Teilmenge von (L) zu (E), vorzugsweise als Teilstrom der die Waschstufe (9) verlassenden organischen Phase (J).

Wegen der erhöhten Kristallisationsneigung der Ammoniumsalze der Polyamine, insbesondere bei Abwesenheit von Hilfsamin, wird die Extraktionsstufe (7) bei Abwesenheit von Hilfsamin in der Regel bei erhöhten Temperaturen betrieben, vorzugsweise bei Temperaturen oberhalb von 80°C, gegebenenfalls unter Druck.

Das zusammen mit der wäßrigen Phase (C) in den Extraktor (7) eingebrachte Ausgangspolyamin (A) verteilt sich auf die den Extraktor verlassende wäßrige Phase (H) und die den Extraktor (7) verlassende organische Phase (D) (quantitative Fraktionierung).

Die mengenmäßige Aufteilung der einzelnen Komponenten des Ausgangspolyamingemisches auf die resultierende wäßrige Phase (H) und die resultierende organische Phase (D) erfolgt unter den Bedingungen des erfindungsgemäßen Verfahrens mit einer überraschend hohen Selektivität, so daß die resultierenden Produktfraktionen eine andere, unter Umständen stark von der des Ausgangspolyamingemisches abweichende Zusammensetzung aufweisen (qualitative Fraktionierung).

Beispielsweise ausgehend von den bevorzugt eingesetzten Anilinformaldehydkondensationsprodukten wurde gefunden, daß von einer in zwei oder mehreren isomeren Formen im Ausgangsgemisch enthaltenen Polyaminkomponenten in der Regel die ortho-Isomere(n) Form(en) in der die Trennstufe (7) verlassenden organischen Phase (D) relativ angereichert ist (sind); beispielsweise 2,4'-Diamino-diphenylmethan relativ zu 4,4'-Diamino-diphenylmethan. Umgekehrt ist die resultierende wäßrige Phase (H) relativ verarmt an dem 2,4'-Isomeren, während das 4,4'-Isomere relativ angereichert ist.

Sind mehrere "ortho-Isomere" im Ausgangspolyamin vorhanden, z.B. 2,2'- und 2,4'-Diamino-diphenylmethan, dann ist das "ortho-reichere" 2,2'-Isomere in der organischen Phase (D) gegenüber dem "ortho-ärmeren" 2,4'-Isomeren stärker angereichert, welchletzteres seinerseits gegenüber dem noch "ortho-ärmeren" 4,4'-Isomeren relativ angereichert ist.

Der zuerst bei den Anilinformaldehydkondensationsprodukten der Diamino-diphylmethanreihe gefundene An- und Abreicherungseffekt wurde rein empirisch deskriptiv mit dem Kriterium der ortho- und para-Subtitution verbunden. Die davon abgeleitete Charakterisierung der Verfahrensprodukte als "orthoreich" und "orthoarm" ist dabei relativ und wurde durch den Begriff "ortho-Substitutionsgrad" ausgedrückt.

Als "ortho-Subtitutionsgrad" wird dabei das Verhältnis der orthoständigen Amlnogruppen-Methylengruppenrelationen zur Gesamtzahl aller Aminogruppenrelationen definiert. Mit diesem Begriff lassen sich praktisch alle Isomerentrennungen bei den Polyaminen erfassen, die aus Arylaminen, auch substituierten, mit Carbonylverbindungen in wäßrig saurem Medium hergestellt werden.

Überraschenderweise wurde nun der gleiche An- und Abreicherungseffekt - geordnet nach ortho-Substitutionsgrad - auch für die gut charakterisierten und analytisch erfaßbaren isomeren Dreikernverbindungen aus der Anilin-/Formaldehydkondensation gefunden.

Analoges gilt für die Trennung von Kondensationsprodukten aus Formaldehyd mit Anilin und Diaminoarylverbindungen wie Phenylendiamin oder alkylsubstituierten Phenylendiaminen.

Die bisher erwähnten Polyamingemische weisen, bedingt durch ihre Herstellung Aminogruppen auf, die praktisch nur orthoständig und/oder paraständig zu Methylengruppen sind.

Dabei werden innerhalb einer Gruppe isomerer Verbindungen in der Regel diejenigen mit den höheren ortho-Substitutionsgrad gegenüber den Isomeren mit einem geringeren ortho-Substitutionsgrad bei der Fraktionierung in der organischen Phase (D) angereichert.

Polyamingemische insbesondere der Diphenylmethanreihe einschließlich der jeweiligen höherkernigen Homologen, die nach anderen Verfahren hergestellt werden, beispielsweise durch Nitrierung von Diphenylmethan oder Methyldiphenylmethanen und anschließende Reduktion, weisen außer ortho- und para-ständigen Aminogruppen herstellungsbedingt auch andere Aminogruppen-Methylengruppenrelationen auf. Für diese Polyamingemische ist das erfindungsgemäße Verfahren genauso wirksam.

Beispielweise läßt sich aus einem Gemisch von 2- und 4-Methyldiphenylmethan durch Nitrierung und anschließende Reduktion ein Polyamingemisch herstellen, welches in der Hauptsache ein Isomerengemisch darstellt aus

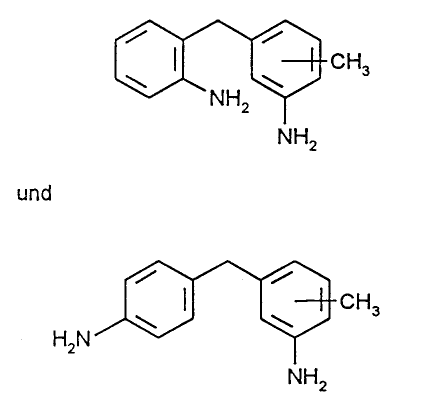

Bei der Fraktionierung solcher Gemische mit Hilfe des erfindungsgemäßen Verfahrens werden die 3,2'-Isomeren in der organischen Phase (D) gegenüber den 3,4'-Isomeren angereichert.

Das Kriterium "orthoreich" und "orthoarm" oder der ortho-Substitutionsgrad erfaßt in diesen Polyamingemischen nicht mehr alle Isomeren und ist daher sinngemäß anzuwenden, indem anstelle der Begriffe "orthoständig" und "paraständig" eine Einteilung der Isomeren vorgenommen wird in solche mit kleinerem (= ortho) und solche mit größerem (= para) räumlichen Abstand der - in der Regel an unterschiedlichen Sechsringen befindlichen - Aminogruppen zur Methylenbrücke bzw. der Aminogruppen zueinander.

Eine weitere Klasse aromatischer Polyamingemische, die sich mit Hilfe des erfindungsgemäßen Verfahrens sehr wirksam fraktionieren lassen, stellen die Polyamine des Triphenylmethans und seiner höherkernigen Homologen, vorzugsweise Benzylhomologen dar, wie sie z.B. durch Nitrierung und anschließende Reduktion der entsprechenden Kohlenwasserstoffgemische hergestellt werden.

Bei der Fraktionierung technischer Polyamingemische der zuletzt genannten Substanzklassen
I. Mischkondensationsprodukte von Mono- und Diaminoarylverbindungen mit Formaldehyd bzw. allgemeinen Carbonylverbindungen,
II. Polyamingemische aus Verfahren durch Nitrierung und anschließende Reduktion von Diphenylmethan und vorzugsweise substituierten insbesondere alkylsubstituierten Diphenylmethanen und den jeweiligen Homologen und
III. Polyamingemische aus Verfahren durch Nitrierung und anschließende Reduktion von Triphenylmethan und vorzugsweise substituierten insbesondere alkylsubstituierten Triphenylmethanen und den jeweiligen höherkernigen Benzylhomologen
wurde zusätzlich zur reinen Isomerentrennung eine weitere überraschende Selektivität gefunden.

Polyamingemische der genannten Substanzklasse I bis III enthalten oder können enthalten Komponenten, bei denen wenigstens ein Arylkern pro Molekül mehr als eine, in der Regel zwei Aminogruppen trägt. Diese Komponenten können die bevorzugten Bestandteile des Polyamingemisches sein, ohne daß sie verfahrensbedingt mengenmäßig die Hauptprodukte sein müssen.

Zur besseren Charakterisierung solcher Komponenten wird der Begriff "Aminosubstitutionsgrad" verwendet, mit dem in erster Linie die Anzahl der Aminogruppen einer Komponente im Verhältnis zur Anzahl der Arylkerne gekennzeichnet wird.

Für Anilin und seine Kondensationsprodukte mit Formaldehyd ist dieser Ausdruck stets 1,0 , für Phenylendiamin und seine Kondensationsprodukte stets 2,0. Für reine Mischkondensate ergeben sich für die Diphenylmethanisomeren der Wert 1,5 und für die höherkernigen Homologen Werte zwischen >1,0 und <2,0. Bei statistischer Verwendung des Begriffs Aminosubstitutionsgrad zur Charakterisierung von technischen Polyamingemischen ergeben sich ebenfalls Werte zwischen 1,0 und 2,0.

Bei der Fraktionierung von Polyamingemischen mit einem Aminosubstitutionsgrad >1,0 wurde nun gefunden, daß die Komponenten mit einem höheren Aminosubstitutionsgrad in der im eigentlichen Trennschritt resultierenden wäßrigen Phase relativ angereichert werden, und zwar um so stärker je größer der Aminosubstitutionsgrad ist.

Somit eröffnet das erfindungsgemäße Verfahren auch für diese Substanzklasse die Möglichkeit, die Herstellungsform der Rohstoffe (Aminstufe) und die Verwendungsform der Endprodukte (Isocyanatstufe) zu entkoppeln, so daß eine getrennte Optimierung beider Stufen erleichtert wird bis hin zur Gewinnung völlig neuer Isocyanatgemische.

Ergänzt werden diese "Leistungen" durch ein weiteres Selektivitätskriterium, welches bei der Fraktionierung technischer Polyamingemische, insbesondere solcher mit höherkernigen Homologen, gefunden wurde und die "Kernigkeit" der Polyamingemische betrifft.

Mit dem Begriff der "Kernigkeit" wird primär die Anzahl der Aryleinheiten einer Komponente eines aromatischen Polyamingemisches ausgedrückt. Im weiten Sinn wird der Begriff der Kernigkeit dafür verwendet, um für ein aus zahlreichen Komponenten, mit einer individuellen exakten Kernigkeit, bestehendes Polyamingemisch statistisch eine Kernigkeit des Gesamtgemisches auszudrücken.

Besonders überraschenderweise wurde nun bei der Fraktionierung von Polyamingemischen mit höherkernigen Anteilen, insbesondere bei der Fraktionierung technischer Gemische von Anilin Formaldehydkondensaten, gefunden, daß sich die höherkernigen Komponenten in der die Fraktionierungsstufe verlassenden organischen Phase gezielt sowohl relativ anreichern als auch relativ abreichern lassen, in Abhängigkeit von der Molarität der wäßrigen Phase in der Extraktionsstufe (7).

Eine hohe Molarität der wäßrigen Phase in (7) innerhalb des angegebenen Molaritätsbereichs führt zu einer relativen Abreicherung höherkerniger Komponenten in der organischen Phase (D) und dementsprechend zu einer relativen Anreicherung in der wäßrigen Phase (H).

Eine niedrige Molarität der wäßrigen Phase in (7) innerhalb des angegebenen Molaritätsbereichs führt zu einer relativen Anreicherung höherkerniger Komponenten in der organischen Phase (D).

Der überraschende Befund kann dahingehend erweitert und präzisiert werden, daß die relative An- und Abreicherung auch innerhalb der höherkernigen Homologen untereinander stattfindet. Werden beispielsweise in einem technischen Gemisch des Diamino-diphenylmethans in der einen Fraktion die Dreikernkomponenten gegenüber den Zweikernkomponenten relativ an- oder abgereichert, wird auch eine relative An- oder Abreicherung von Vierkernkomponenten gegenüber Dreikernkomponenten, d.h. eine noch stärkere relative An- oder Abreicherung, gefunden, desgleichen von Fünfkernkomponenten gegenüber Vierkernkomponenten usw.

Daraus und aus der gleichzeitig und stets im Sinne einer relativen Verstärkung des "ortho-Substitutionsgrades" in der organischen Phase (D) ablaufenden Isomerentrennung und aus der Möglichkeit, mit einzelnen Produktfraktionen die erfindungsgemäße Trennung, gegebenenfalls mit geänderten Verfahrensparametern, zu wiederholen, ergeben sich zahlreiche Möglichkeiten, ausgehend von bekannten und gut zugänglichen Polyamingemischen über das erfindungsgemäße Verfahren zu weniger gut zugänglichen oder völlig neuen, weil nach dem Stand der Technik bislang unzugänglichen, Polyaminen und damit Polyisocyanaten zu gelangen. Das gilt besonders für Produkte der Diamino- und Diisocyanato-diphenylmethanreihe und ganz besonders für Polyamin- und Polyisocyanatgemische mit einem extrem hohen Anteil an höherkernigen Komponenten.

Die An- bzw. Abreicherung wird in der Regel effektiver mit steigendem Protonierungsgrad in der wäßrigen Phase der Trennstufe.

Darüber hinaus erweist sich das erfindungsgemäße Verfahren als von allgemeiner Wirksamkeit auch auf andere strukturähnliche Polyamine.

So können beispielsweise in den bereits erwähnten Polyamingemischen, die durch Nitrierung von Di- und Polyarylmethanen und anschließender Reduktion gewonnen werden, auch Monoaminopolyarylmethanverbindungen oder Komponenten enthalten sein, bei denen eine oder mehrere Methylengruppen durch Nebenreaktionen in Keto- und/oder Hydroxymethylengruppen und damit in unerwünschte Nebenprodukte umgewandelt worden sind.

Bei der Kondensation von Arylaminen mit Carbonylverbindungen können zahlreiche unvollständig umgelagerte Zwischenverbindungen und Nebenprodukte auftreten.

Die meisten dieser Verbindungen unterliegen in der Regel bei der Fraktionierung der sie enthaltenen Polyamingemische einer Anreicherung in einer der resultierenden Fraktionen, so daß der Effekt zur Abtrennung und Fraktionierung genutzt werden kann.

Gegebenenfalls können derartige Produkte auf diesem Weg angereichert werden oder als gezielt hergestellte Polyamingemische, wie z.B. Polyaminobenzophenone oder Aminobenzylarylamingemische ihrerseits fraktioniert werden.

Die die Extraktionsstufe (7) verlassende, organische Phase (D) enthält unter anderem noch geringe Mengen an Säure, im allgemeinen und in Abhängigkeit von den Verfahrensparametern in den Extraktionsstufen (6) und (7) zwischen 0,01 und 0,5 Gew.-%, die vorteilhaft vor der destillativen Aufarbeitung des Mengenstromes (D) entfernt werden.

Im einfachsten Fall geschieht dies durch Neutralisation mit überschüssigen, verdünnten wäßrigen Basen, beispielsweise verdünnter Natronlauge.

Die organische Phase (D) wird zumindest teilweise gegebenenfalls nach Durchlaufen der Waschstufe (10), in die Destillationsstufe (11) überführt.

In der letzten Stufe der gegebenenfalls mehrstufigen Destillationsstufe (11) wird das erste Polyaminteilprodukt (G) abgetrennt und im Verfahrensprodukttank (13) gesammelt.

Das entsprechende zweite Teilprodukt befindet sich in der die Extraktionsstufe (7) verlassenden wäßrigen Phase (H).

Die wäßrige Phase (H) wird gegebenenfalls nach Zusatz von Hilfsamin und/oder hydrophobem Lösungsmittel, in der Neutralisationsstufe (8) mit der wäßrigen Lösung einer starken Base, vorzugsweise konz. Natronlauge, zur Neutralisation der enthaltenen Säure umgesetzt.

Die bei der Neutralisation gebildete wäßrige Phase wird abgetrennt und im Abwasserbehälter (15) gesammelt.

Die bei der Neutralisation gebildete organische Phase wird als Mengenstrom (J) abgetrennt und zumindest teilweise, gegebenenfalls in der Waschstufe (9) mit Wasser gewaschen, und der destillativen Aufarbeitung (12) zugeführt.

In der letzten Stufe der gegebenenfalls mehrstufigen Destillationsstufe (12) wird das zweite Polyaminteilprodukt (L) abgetrennt und im Verfahrensprodukttank (14) gesammelt.

Mit dieser ersten Variante des erfindungsgemäßen Verfahrens lassen sich beträchtlich Trennleistungen bei der Fraktionierung von Polyamingemischen erzielen und zahlreiche Trennprobleme zufriedenstellend lösen.

Insbesondere in der zweiten Polyaminfraktion (L) kann die relative Anreicherung der in dieser Fraktion bevorzugt enthaltenen Komponente gezielt variiert und maximiert werden.

Der in der ersten Polyaminfraktion (G) verbleibende Anteil dieser Komponenten kann jedoch gemäß dieser ersten Variante nicht in gleicher Weise minimiert werden, sondern variabel nur bis zu einem Gehalt relativ abgereichert werden, dessen untere Grenze abhängt von dem für die jeweiligen Verfahrensparameter charakteristischen Verteilungsgleichgewichte der Polyaminkomponenten von (A) zwischen der organischen Phase (D) beim Verlassen des Extraktors (7) und der wäßrigen Phase (C) beim Eintritt in den Extraktor (7).

Die organische Phase (B) besteht im allgemeinen neben hydrophobem Lösungsmittel gegebenenfalls aus Hilfsamin und/oder Polyamin, letzteres vorzugsweise mit der Zusammensetzung des zweiten Verfahrensteilproduktes (L).

Bei der Verwendung einer organischen Phase (B) ohne Polyamin resultiert in der die Extraktionsstute (7) verlassenden wäßrigen Phase (H) eine Polyaminfraktion, in welcher die relative Anreicherung der in dieser Phase bevorzugt enthaltenen Komponenten gezielt erhöht und maximiert werden kann, auf Kosten der Polyaminkonzentration in der wäßrigen Phase.

Polyamin als Bestandteil der organischen Phase (B) bewirkt, daß die die Verfahrensstufe (7) verlassende Phase (H) eine höhere und damit für die Durchführung des erfindungsgemäßen Verfahrens energetisch vorteilhaftere Polyaminkonzentration aufweisen, als bei Verwendung einer organischen Phase (B) ohne Polyamin.

Durch die bevorzugte Verwendung eines Polyamins mit der Zusammensetzung des zweiten Teilproduktes (L) als Bestandteil der organischen Phase (B) kann auch auf einem höheren und damit vorteilhaften Konzentrationsniveau infolge Gleichgewichtseinstellung mit Selbstverstärkung des Trenneffektes die relative Anreicherung der in der die Trennstufe (7) verlassenden wäßrigen Phase (H) bevorzugt enthaltenen Polyaminkomponeten und damit der zweiten Polyaminfraktion (L) variiert und maximiert werden.

Vorteilhafter und als Ausführungsform bevorzugt ist eine zweite Variante des erfindungsgemäßen Verfahrens, bei der sich zusätzlich auch in der ersten Polyaminfraktion (G) die relevante Anreicherung der in dieser Fraktion bevorzugt enthaltenen Komponenten beträchtlich steigern und gezielt variieren läßt, indem die in der Extraktionsstufe (7) anfallende organischen Phase (D) zumindest teilweise in einer aus der Sicht der Phase (D) zwischengeschalteten Extraktionsstufe (6) mit einer wäßrigen Phase extrahiert wird, die im vorliegendem Fall der Variante 2 im wesentlichen aus zumindest einer Teilmenge des Mengenstroms (X) und gegebenenfalls zusätzlichem Wasser aus Mengenstrom (Y) besteht.

Aus formalen Gründen wird die der Extraktionsstufe (6) zugeführte organische Phase als Mengenstrom (M) bezeichnet, auch wenn sie gegebenenfalls wie im vorliegenden Fall beispielhaft ausgeführt, zumindest in der Zusammensetzung, vorzugsweise aber auch in der Menge mit Mengenstrom (D) übereinstimmt.

Bereits bei der einstufigen Durchführung der Extraktionsstute (6), beispielsweise als Mischer-Scheidereinheit, kommt es in der resultierenden organischen Phase (O) in Abhängigkeit von Art und insbesondere Menge der eingesetzten wäßrigen Phase zu einer deutlichen weiteren relativen Anreicherung der bereits in (D) gegenüber Ausgangspolyamin (A) angereicherten Komponenten, verbunden mit einer Abnahme des Polyamingehaltes in der resultierenden organischen Phase (O). Vorzugsweise wird jedoch wegen der besseren Effektivität auch die zwischengeschaltete Extraktionsstufe (6) als mehrstufig wirkender und im Gegenstrom betriebener Extraktor ausgeführt.

Die in der Extraktionsstufe (6) anfallende wäßrige Phase (N) enthält die entsprechende andere Fraktion des mit Mengenstrom (M) eingebrachten Polyamins, in der die in (O) angereicherten Komponenten entsprechend abgereichert sind. Ausmaß der relativen Abreicherung, d.h. die Zusammensetzung des in (O) enthaltenen Polyamins, wird unter den jeweiligen Verfahrensbedingungen der mehrstufig wirkenden Extraktionsstufe (6) kontrolliert durch das qualitative und quantitative Verteilungsgleichgewicht zwischen der zugeführten organischen Phase (M) und der abgeführten wäßrigen Phase (N).

Die Molarität der wäßrigen Phase in der Extraktionsstufe (6) liegt je nach Trennaufgabe höher oder auf gleicher Höhe oder niedriger bezogen auf die Molarität in der aus Sicht der wäßrigen Phase nachgeschalteten Extraktionsstufe (7) und wird durch die Zugabe von Säure und/oder die Zugabe oder gegebenenfalls den Entzug von Wasser an geeigneter Stelle geregelt.

Die in der Verfahrensstufe (6) resultierende wäßrige Phase (N) wird gegebenenfalls nach Zugabe von Wasser zusammen mit dem gegebenenfalls vorhandenen Rest von (X) der Extraktionsstufe (7) zugeführt.

Die in Stufe (6) resultierende organische Phase (O) wird zusammen mit dem gegebenenfalls vorhandenen Rest von (D) der Destillationsstufe (11) zugeführt zur Gewinnung der Polyaminfraktion (G).

Mit der zweiten Variante des erfindungsgemäßen Verfahrens läßt sich die relative Anreicherung in beiden resultierenden Polyaminfraktionen gezielt variieren und maximieren. Neben dieser in qualitativer Hinsicht großen Vielseitigkeit und Leistungsfähigkeit stellt die zweite Verfahrensvariante zumindest für die zweite Polyaminfraktion (L) auch eine energetisch günstige Ausführungsform dar.

Der mit Gewinnung der ersten Polyaminfraktion (G) verbundene Energieaufwand steigt dagegen relativ um so stärker, je geringer der mengenmäßige Anteil von (G), bezogen auf eingesetztes Polyamingemisch (A) ist, weil der verbleibende Gehalt an Polyamin (G) in der destillativ aufzuarbeitenden organischen Phase (D) bzw. (O) gegebenenfalls immer kleiner wird.

Eine in dieser Hinsicht verbesserte Ausführung stellt die dritte Variante des erfindungsgemäßen Verfahrens dar. Ausgehend von der ersten Variante wird diese dahingehend erweitert, daß die die Verfahrensstufe (7) verlassende, das erste Teilprodukt (G) in gegenüber der Konzentration von (A) in (C) verringerter Konzentration enthaltende organische Phase (D) geteilt wird in einen Mengenstrom (D'), der weiterhin mit dem Ziel der Gewinnung der Polyaminfraktion (G) den Aufarbeitungsstufen (10) und (11) zugeführt wird, und in einen Mengenstrom (P).

Der Mengenstrom (P) wird in einer vorgelagerten Extraktionsstufe (5) mit zumindest einer Teilmenge, vorzugsweise mit der Gesamtmenge der als Mengenstrom (X) zur Verfügung stehenden wäßrigen Säure umgesetzt; gegebenenfalls erfolgt die Umsetzung als mehrstufige Gegenstromextraktion.

Bei der Extraktionsstufe (5) handelt es sich in der Regel um einen mehrstufig wirkenden und im Gegenstrom betriebenen Extraktor, in welchem die zugeführte organische Phase (P) mit zumindest einer Teilmenge, vorzugsweise mit der Gesamtmenge der zur Wiederverwendung zur Verfügung stehenden wäßrigen Säure (X) extrahiert wird.

Der dem Extraktor (5) zugeführte Mengenstrom (P) wird dabei so bemessen, daß bei der Umsetzung mit Mengenstrom (X) ein möglichst weitgehender, vorzugsweise praktisch quantitativer Übergang zumindest der in der organischen Phase (P) enthaltenen Polyamine in die den Extraktor (5) verlassende wäßrige Phase (Q) erfolgt.

Der Restgehalt an Polyamin in der die Verfahrensstufe (5) resultierenden organische Phase (R) liegt im allgemeinen bei <3 Gew.-%, vorzugsweise bei <1 Gew.-%.

Im übrigen richtet der sich in (R) zulässige Höchstgehalt an Amin und insbesondere der Gehalt an Polyamin nach den aus der jeweiligen Trennaufgabe resultierenden qualitativen Anforderungen an die Verfahrensprodukte, im Falle der Variante 3 insbesondere an das Verfahrensteilprodukt (L). Die Einhaltung des für die Qualität von (L) relevanten Gehaltes an Polyamin wird im Rahmen der technischen Gegebenheiten unter Ausschöpfung der zur Verfügung stehenden wäßrigen Säure (X), gegebenenfalls einer Menge von (Y), über die Bemessung des Teilmengenstromes (P) kontrolliert.

Dabei kommt es dem Verfahren und insbesondere der Extraktionsstufe (5) zustatten, daß die für den Einsatz in Stufe (5) zur Verfügung stehende wäßrige Säure (Mengenstrom X) um so größer ist, je größer der Anteil der zweiten Polyaminfraktion (L) und je kleiner demzufolge der Anteil der ersten Polyaminfraktion (G) ist. Eine kleine Polyaminfraktion (G) bedeutet in der Regel eine niedrige Polyaminkonzentration in der organischen Phase (D) und hohen Energieaufwand bei der Aufarbeitung einer solchen Phase. Durch die erfindungsgemäße Variante 3 kann gegenüber Variante 1 insbesondere der Energieaufwand bei der Isolierung der ersten Polyaminfraktion (G) reduziert werden.

Der Beitrag der Verfahrensstufe (5) im Rahmen der Variante 3 zur Verbesserung des erfindungsgemäßen Verfahrens besteht darin, daß für die destillative Aufarbeitung (11) zur Gewinnung der ersten Polyaminfraktion (G) anstelle des Gesamtstromes mit einer relativ niedrigen und damit energetisch ungünstigen Konzentration an Polyamin nur ein Teilstrom mit entsprechend erhöhter und damit energetisch günstigerer Konzentration anfällt (quantitative Anreicherung), während aus dem anderen Teilstrom eine an geeigneter Stelle als Extraktionsmittel verwendbare organische Phase (R) ohne Destillation gewonnen wird.

Die von Polyamin weitgehend befreite, die Verfahrensstufe (5) verlassende organische Phase (R) wird der Extraktionsstufe (7) zugeführt.

In der vorzugsweise mehrstufig wirkenden Extraktionsstufe (7) wird die organische Phase (R) als Extraktionsmittel zugesetzt, in der Regel durch Vermischen mit Mengenstrom (B) und Zugabe zu der aus der Sicht der organischen Phase (B) ersten Stufe des Extraktors.

In Abhängigkeit von einem gegebenenfalls vorhandenen Restgehalt an Polyamin in (R) und mit Rücksicht auf die Qualität der zweiten Polyaminfraktion (L) erfolgt die Zugabe der organischen Phase (R) gegebenenfalls zu einer aus Sicht der organischen Phase (B) späteren, gegebenenfalls zur letzten Stufe der mehrstufig arbeitenden Extraktionsstufe (7).

Gegebenenfall erfolgt die Zugabe von (R) zumindest teilweise zu der gegebenenfalls vorhandenen vorgeschaltenen Stufe (7A), die in diesem Falle vorzugsweise als Mischer Scheidereinheit betrieben wird. Nach der Auftrennung des im Mischer gebildeten zweiphasigen Gemisches wird gegebenenfalls nur die wäßrige Phase der Verfahrensstufe (7) zugeführt, die abgetrennte organische Phase dagegen wird dem Teilstrom von (D) zugeschlagen, der den Extraktionsstufen (5) und/oder (6) zugeführt wird.

Die die Verfahrensstufe (5) verlassende wäßrige Phase (Q) enthält neben der zumindest teilweise in Form ihrer Ammoniumsalze vorliegenden Säure Polyamin mit einer Zusammensetzung, die weitgehend dem Polyamin in der zugeführten organischen Phase (P) entspricht und gegebenenfalls Hilfsamin.

Im Falle der Variante 3 des erfindungsgemäßen Verfahrens wird der Mengenstrom (Q) direkt der Verfahrensstufe (7) zugeführt, gegebenenfalls nach Zugabe von Wasser aus Mengenstrom (Y) und/oder weiterer wäßriger Säure aus Mengenstrom (X).

Da die in der wäßrigen Phase (Q) enthaltene Polyaminfraktion in der Regel eine höhere relative (qualitative) Anreicherung im Sinne der ersten Polyaminfraktion (G) bezogen auf das Ausgangspolyamin (A) aufweist, resultiert für die der Extraktionsstufe (7) zugeführte wäßrige Phase nach Zugabe von Ausgangspolyamin (A) ein gegenüber diesem in Abhängigkeit vom Mengenverhältnis "angereichertes" Mischpolyamin. Infolge des Verteilungsgleichgewichtes zwischen zugeführter wäßriger und resultierender organischer Phase (D) ergibt daraus für Variante 3 auch ein begrenzter zusätzlicher qualitativer Anreicherungseffekt für die erste Polyaminfraktion (G).

In einer weiteren Variante 4 des erfindungsgemäßen Verfahrens werden die technischen Maßnahmen der vorausgegangenen Varianten zusammengefaßt und miteinander kombiniert.

Im einfachsten Fall werden die Extraktionsstufen (5) und (6) hinzugefügt und jede für sich mit einem Teilstrom von (X), gegebenenfalls einen Teilstrom von (Y) und einem Teilstrom von (D), der in diesem Fall gegebenenfalls in drei Teilströme aufgeteilt wird, in der beschriebenen Weise durchgeführt.

Vorteilhafter ist es, als organische Phase (P) in Extraktionsstufe (5) einen Teilstrom des, eine qualitativ hochangereicherte und quantitativ weniger konzentrierte Polyaminfraktion enthaltenden, Mengenstromes (O) einsetzen.

Bevorzugt wird die Variante 4 so durchgeführt, daß als organische Phase (P) in der Extraktionsstufe (5) ein Teilstrom von (D) und/oder vorzugsweise ein Teilstrom von (O) eingesetzt wird und die in Stufe (5) resultierende wäßrige Phase (Q) zumindest teilweise, vorzugsweise insgesamt der Extraktionsstufe (6) zugeführt und gegebenenfalls unter Zugabe von weiterer wäßriger Säure aus Mengenstrom (X) und gegebenenfalls von Hilfsamin in (6) eingesetzt wird. Dabei wird ihr unter inniger Durchmischung in mehreren Stufen die organische Phase (M) mit ihrem Gehalt an im gleichen Sinne angereicherten Polyamin entgegengeführt, gegebenenfalls wird die organische Phase (M) verstärkt durch Zugabe eines Teilstromes der in der Extraktionsstufe (5) resultierenden organischen Phase (R) zu (M).

Durch diese Maßnahmen kommt es in der in (6) resultierenden organischen Phase (O) zu einer weiteren Steigerung des qualitativen Anreicherungseffektes. In quantitativer Hinsicht kann dieses Ergebnis durch Bemessung und Aufteilung der Mengenströme bei einem relativ hohen und damit energetisch günstigem Polyamingehalt in den in (6) resultierenden Phasen, insbesondere in der organischen Phase (O) erreicht werden.

Die Rückkopplung des Anreicherungseffektes in (O) über den Mengenstrom (P) als Teilstrom von (O) und über die wäßrige Phase (Q) wirkt dabei selbstverstärkend.

Durch die erfindungsgemäße Ausführung und Verschaltung der Extraktionsstufe (5) bis (7) in Variante (4) mit Verfahrenskriterien wie Disproportionierung anstelle von fraktionierter Extraktion in Stufe (6), mit Selbstverstärkung durch Verschaltung mit Extraktionsstufe (5) und Wiedergewinnung von Extraktionsmittel in Stufe (5) ohne Destillation für den Einsatz in der Verfahrensstufe (7) und gegebenenfalls in (6) resultiert ein Maximum an qualitativer Trennleistung, die in Kombination mit der Variation der Molarität der wäßrigen Phasen in den Stufen (5) bis (7) zu einer großen Anwendungsbreite des erfindungsgemäßen Verfahrens führt.

### Beispiele

### Beispiel 1

In einem Mischer (7A) werden das Ausgangspolyamingemisch (Mengenstrom A) (1,260 kg/h) mit Anilin (0,495 kg/h) (Teilmengenströme F und K), 30 %iger Salzsäure (0,885 kg/h) (Mengenstrom X) und Wasser (1,860 kg/h) (Mengenstrom Y) miteinander vermischt unter Bildung des wäßrigen Mengenstrom (C).

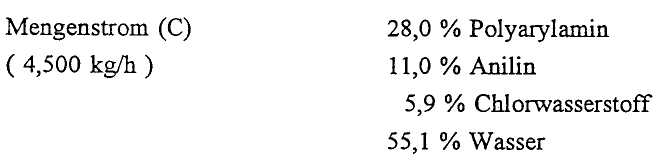

Mengenstrom (C) wird in einen mehrstufig wirkenden Extraktor bei 90°C dem organischen Mengenstrom (B) entgegengeführt.

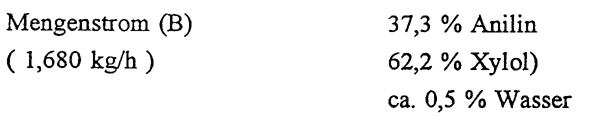

Die dabei resultierende, die Extraktionsstufe (7) verlassende organische Phase (D) bat folgende durchschnittliche Zusammensetzung:

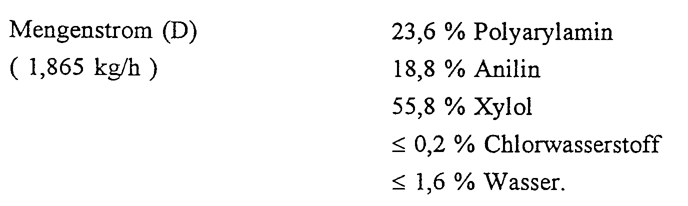

Mengenstrom (D) wird in der Neutralisationsstufe (10) mit überschüssiger verdünnter Natronlauge (Teilmengenstrom von Z und Wasser aus 2) gewaschen. Die wäßrige Phase wird als Abwasser in Tank (15) gesammelt.

Der gewaschene und von Säureresten befreite Mengenstrom (D) wird in einer ersten Destillationsstufe (11.1) weitgehend vom Xylol und einem Teil des Anilins und in einer zweiten Destillationsstufe (11.2) von dem verbliebenen Anilin und restlichen Xylol befreit. Die beiden Destillate (Mengenstrom E und F) werden vereinigt und bilden zusammen mit einem entsprechenden Teilstrom von (K) den Mengenstrom (B).

Der in Destillationsstufe (11) resultierende Rückstand besteht aus Polyamingemisch, welches als Mengenstrom (G) mit 0,441 kg/h in Tank (13) gesammelt wird.

Die den Extraktor (7) verlassende wäßrige Phase (H) hat folgende durchschnittliche Zusammensetzung

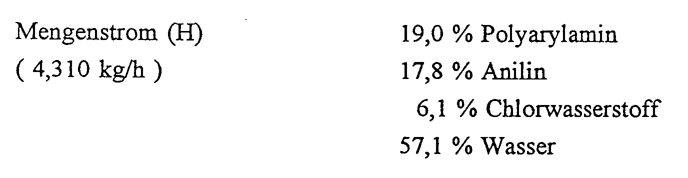

und wird in der Neutralisationsstufe (8) mit überschüssiger wäßriger Natronlauge aus Tank (3), (Teilmengenstrom Z) neutralisiert. Die wäßrige, salzhaltige Phase wird abgetrennt und im Abwassertank (15) gesammelt.

Die organische Phase wird anschließend in der Waschstufe (9) mit Wasser aus Tank (2) weitgehend salzfrei gewaschen. Das Waschwasser wird ebenfalls im Abwassertank (15) gesammelt.

Die, die Waschstufe verlassende, organische Phase (Mengenstrom J) wird in der Destillationsstufe (12) aufgetrennt in eine Destillatfraktion (Mengenstrom K) und einen Destillationsrückstand (Mengenstrom L).

Mengenstrom (K) wird aufgeteilt in einen Teilstrom (0,495 kg/h), welcher den Mischer (7A) zugeführt wird und einen zweiten Teilstrom (0,274 kg/h), welcher zusammen mit den Destillaten (E) und (F) von Destillationsstufe (11) den Mengenstrom (B) bildet.

Der Destillationsrückstand von (12) bildet die zweite Polyaminfraktion (L) mit 0,819 kg/h und wird im Tank (14) gesammelt.

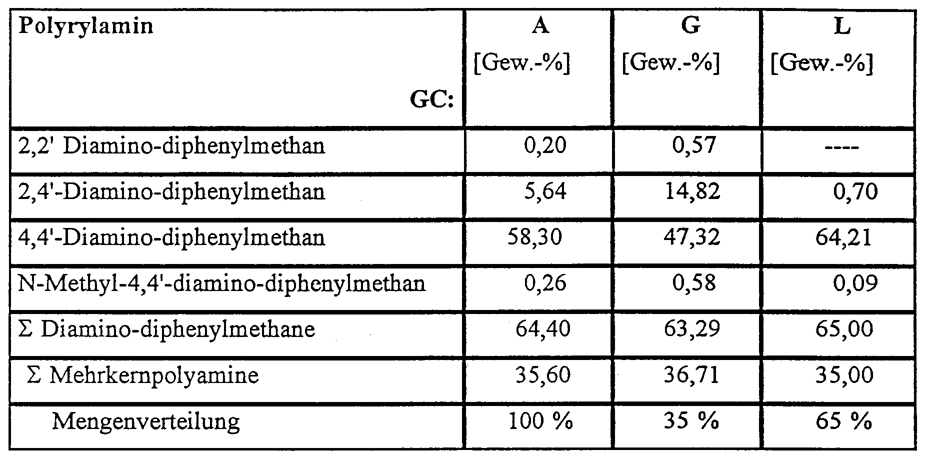

### Beispiel 2

In einem Mischer (7A) werden 1,040 kg/h Ausgangspolyamingemisch (Mengenstrom A) mit 0,319 kg/h Anilin (Teilmengenstrom von K), 0,685 kg/h 30 %iger Salzsäure (Teilmengenstrom von X) und 1,454 kg/h Wasser (Teilmengenstrom von Y) und der wäßrige Mengenstrom (N) miteinander vermischt unter Bildung des wäßrigen Mengenstromes (C).

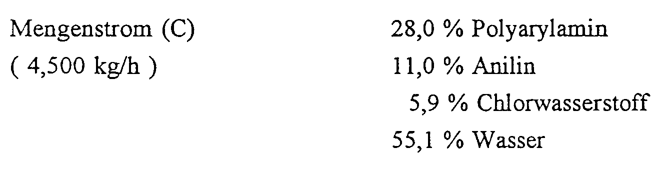

Mengenstrom (C) wird in einen mehrstufig wirkenden Extraktor (7) bei 90°C einer organischen Phase entgegengeführt, die aus den Mengenströmen (B) und (R) gebildet wird.

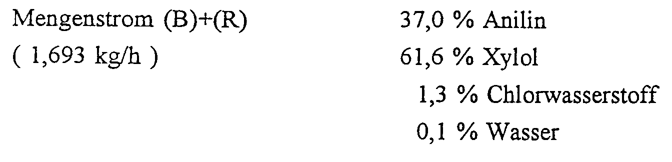

Die dabei resultierende, die Extraktionsstufe (7) verlassende, organische Phase (Mengenstrom D) hat folgende Zusammensetzung

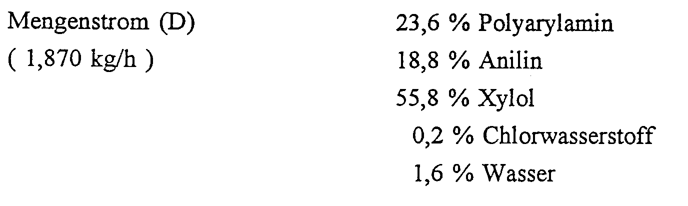

und wird zu gleichen Teilen aufgeteilt in einem Mengenstrom (M) (0,935 kg/h), welcher der Extraktionsstufe (6) zugeführt wird und in einen Mengenstrom (P), welcher der Extraktionsstufe (5) zugeführt wird.

Mengenstrom (P) wird in der mehrstufig wirkenden Extraktionsstufe (5) bei 70°C einer wäßrigen Phase entgegengeführt,die aus den Teilmengenströmen von (X) (0,200 kg/h 30%ige Salzsäure) und (Y) (0,100 kg/h Wasser) gebildet wird.

Die bei diesen Vorgang resultierende, Stufe (5) verlassende, Phase (R) (0,538 kg/h) ist praktisch Aminfrei und besteht im wesentlichen aus Xylol neben bis zur Sättigung gelösten oder mitgerissenen geringen Mengen an Chlorwasserstoff und Wasser.

Die in (5) resultierende wäßrige Phase (Q) hat folgende durchschnittliche Zusammensetzung

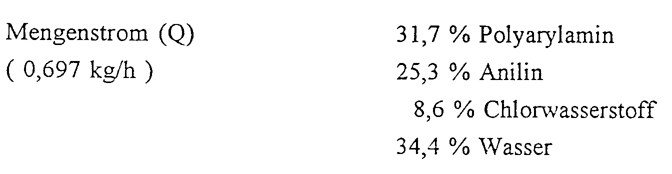

und wird nach Zugabe von 0,305 kg/h als Teilmenge von Mengenstrom (Y) zum Mengenstrom (Q) der mehrstufig wirkenden Extraktionsstufe (6) zugeführt und dort als wäßrige Phase bei 90°C dem Mengenstrom (M) als organischer Phase entgegengeführt.

Die in der Extraktionsstufe (6) resultierende organische Phase (O) hat folgende durchschnittliche Zusammensetzung:

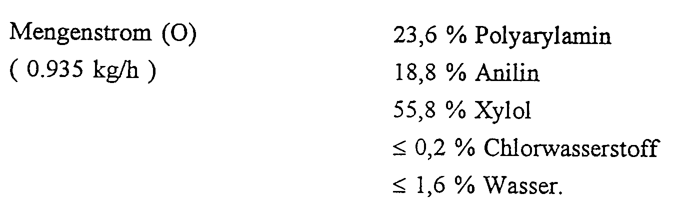

Mengenstrom (O) wird in der Neutralisationsstufe (10) mit überschüssiger verdünnter Natronlauge (Teilmengenstrom von Z und Wasser aus 2) gewaschen. Die wäßrige Phase wird als Abwasser in Tank (15) gesammelt.

Der gewaschene und von Säureresten befreite Mengenstrom (O) wird in einer Destillationsstufe (11) (ein- oder zweistufig) von Wasser, Xylol und Anilin befreit.

Der in Destillationsstufe (11) resultierende Rückstand besteht aus Polyamingemisch, welches als Mengenstrom (G) mit 0,221 kg/h in Tank (13) gesammelt wird.

Das Destillat aus Stufe (11) (0,705 kg/h) wird mit einer Teilmenge (0,450 kg/h) des Destillatstromes (K) aus Destillationsstufe (12) vereinigt unter Bildung von Mengenstrom (B)

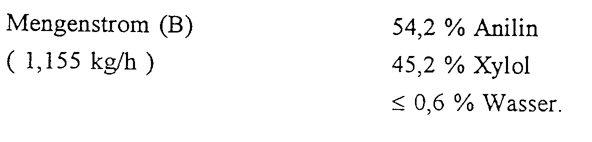

Die den Extraktor (6) verlassende wäßrige Phase (N) wird dem Mischer (7A) zugeführt

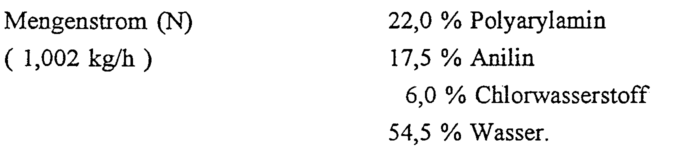

Die in der Extraktionsstufe (7) resultierende wäßrige Phase (H) hat folgende durchschnittliche Zusammensetzung

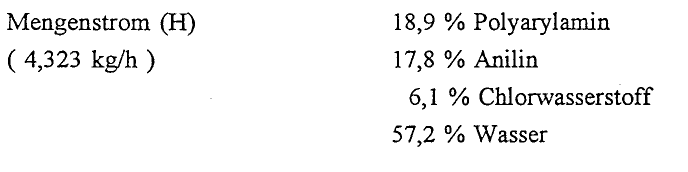

und wird in der Neutralisationsstufe (8) mit überschüssiger wäßriger Natronlauge aus Tank (3), (Teilmengenstrom Z) neutralisiert. Die wäßrige, salzhaltige Phase wird abgetrennt und im Abwassertank (15) gesammelt.

Die organische Phase aus der Neutralisationsstufe (8) wird anschließend in der Waschstufe (9) mit Wasser aus Tank (2) weitgehend salzfrei gewaschen. Das Waschwasser wird ebenfalls im Abwassertank (15) gesammelt.

Die salzfrei gewaschene organische Phase (Mengenstrom J) wird in der Destillationsstufe (12) aufgetrennt in eine Destillatfraktion (Mengenstrom K) und einen Destillationsrückstand (Mengenstrom L).

Mengenstrom (K) wird aufgeteilt in einen Teilstrom (0,319 kg/h), welcher den Mischer (7A) zugeführt wird und einen zweiten Teilstrom (0,450 kg/h), welcher zur Bildung von Mengenstrom (B) verwendet wird.

Der Destillationsrückstand von (12) bildet die zweite Polyaminfraktion (L) mit 0,819 kg/h und wird im Tank (14) gesammelt.

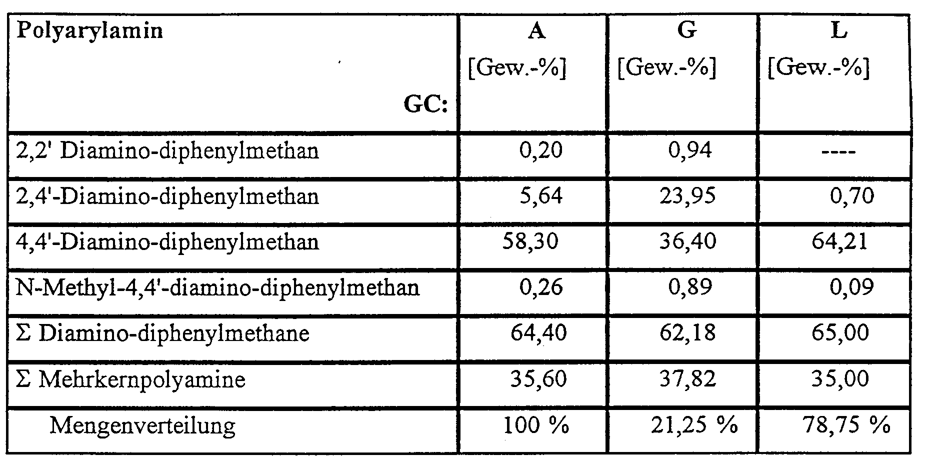

## Patentansprüche

1. Verfahren zur Fraktionierung und Reinigung von aromatischen Polyamingemischen der Diphenylmethanzeihe, dadurch gekennzeichnet, daß man
a) das Polyaminausgangsgemisch (A) in einem zweiphasigen System, bestehend aus (i) einer hydrophoben Lösungsmittelphase (B), die im wesentlichen aus hydrophobem Lösungsmittel besteht, und (ii) einer wäßrigen Phase (C), bestehend im wesentlichen aus Wasser, einer starken Säure und Hilfsamin, welches in Wasser praktisch unlöslich ist und unter Normaldruck einem mindestens 20°C unter dem Siedepunkt der am niedrigsten siedenden Komponente des Ausgangsgemisches und mindestens 20°C oberhalb des Siedepunktes des Lösungsmittels liegenden Siedepunkt aufweist, unter Zuhilfenahme einer nach dem Gegenstromprinzip arbeitenden Extraktionsstufe (7) unter Durchmischung der Phasen verteilt, indem man das Ausgangspolyamingemisch über die wäßrige Phase in die Extraktionsstufe (7) einbringt mit der Maßgabe, daß in diesem zweiphasigen System die Summe der in den Mengenströmen (A), (B) und (C) eingebrachten Aminäquivalente die Anzahl der in Mengenstrom (C) eingebrachten Säureäquivalente stets übersteigt, die diese Extraktionsstufe (7) verlassende organische Phase (D)
b) zumindest teilweise über eine zwischengeschaltete Extraktionsstufe (6) und/oder
c) unter Abtrennung eines Teilstromes vor oder nach der durchlaufenden Extraktionsstufe (6) und Rückführung des abgetrennten Teilstromes zur Extraktionsstufe (7) und/oder zur Extraktionsstufe (6) über eine vorgeschaltete Extraktionsstufe (5)
d) in einer mehrstufigen Destillation (11.1), (11.2) in eine erste, in der Extraktionsstufe (7) wiederverwendete Fraktion (E), bestehend im wesentlichen aus hydrophobem Lösungsmittel, eine zweite Fraktion (F), bestehend im wesentlichen aus Hilfsamin und einem Destillationsrückstand (G), bestehend im wesentlichen aus einer ersten Polyaminfraktion, auftrennt und
e) die die Extraktionsstufe (7) verlassende wäßrige Phase (H) in eine Neutralisationsstufe (8) leitet, mit Basen, die in der wäßrigen Phase enthaltene Säure neutralisiert und anschließend in einem Phasentrennschritt in eine wäßrige Phase, enthaltend die Säure in Form ihrer neutralen Salze und eine organische Phase, enthaltend im wesentlichen Polyamin, mechanisch auftrennt und
f) die in der Neutralisationsstufe (8) anfallende organische Phase (J) nach Durchlaufen eine Waschstufe (9), zumindest teilweise in einer Destillationsstufe (12) aufarbeitet in eine Destillationsfraktion (K), enthaltend die in (J) enthaltenen Anteile an hydrophobem Lösungsmittel, und in eine als Destillationsrückstand (L) anfallende zweite Polyaminfraktion.

2. Verfahren gemäß Anspruch 1, dadurch gekennzeichnet, daß man
b) die in der Extraktionsstufe (7) anfallende organische Phase (D) zumindest teilweise in einer zwischengeschalteten Extraktionsstufe (6) mit zumindest einer Teilmenge, der wäßrigen Säure (Mengenstrom X) und/oder Wasser aus Mengenstrom (Y) und/oder Hilfsamin im Gegenstrom extrahiert und/oder mit der Gesamtmenge der in der vorgeschalteten Extraktionsstufe (5) anfallenden wäßrigen Phase (Q) im Gegenstrom extrahiert, die in der zwischengeschalteten Extraktionsstufe (6) resultierende wäßrige Phase (N) der Extraktionsstufe (7) zuführt und die in der zwischengeschalteten Extraktionsstufe (6) anfallende organische Phase (O) der Aufarbeitungsstufe (11) zuführt.

3. Verfahren gemäß Anspruche 1 bis 2, dadurch gekennzeichnet, daß man
c) einen Teilstrom der die Extraktionsstufe (7) verlassenden organischen Phase (D) und/oder
einen Teilstrom der die zwischengeschaltete Extraktionsstufe (6) verlassenden organischen Phase (O) abtrennt
und in einer vorgeschalteten Extraktionsstufe (5) mit einer Teilmenge, der als Mengenstrom (X) zur Verfügung stehenden wäßrigen Phase im Gegenstrom extrahiert,
den in der Extraktionsstufe (5) eingesetzten organischen Mengenstrom (P) so bemißt, daß in (5) ein möglichst weitgehender Übergang des im besagtem organischen Mengenstrom (P) enthaltenen Polyamins in die wäßrige Phase (Q) erfolgt,
die in der vorgeschalteten Extraktionsstufe (5) resultierende wäßrige Phase (Q) nach Zugabe von Wasser aus Mengenstrom (Y) und/oder Hilfsamin der Extraktionsstufe (6) zuführt und
die in der vorgeschalteten Extraktionsstufe (5) anfallende an Polyamin verarmte organische Phase (R) der Extraktionsstufe (7) und/oder der Extraktionsstufe (6) zuführt.

4. Verfahren gemäß einem oder mehreren der Ansprüche 1 bis 3, dadurch gekennzeichnet, daß man als Hilfsamin Anilin verwendet.

5. Verfahren gemäß einem oder mehreren der Ansprüche 1 bis 3, dadurch gekennzeichnet, daß man als Hilfsamin 2,6-Dimethylanilin verwendet.

6. Verfahren gemäß einem oder mehreren der Ansprüche 1 bis 3, dadurch gekennzeichnet, daß man als Hilfsamin Xylidingemische verwendet.

7. Verfahren gemäß einem oder mehreren der Ansprüche 1 bis 3, dadurch gekennzeichnet, daß man als Hilfsamin 2-Methyl-6-ethylanilin verwendet.

8. Verfahren gemäß einem oder mehreren der Ansprüche 1 bis 7, dadurch gekennzeichnet, daß man als Polyamingemisch der Diphenylmethanreihe ein Polyamingemisch verwendet, wie es bei der säurekatalysierten Anilin/Formaldehyd-Kondensation anfällt.

9. Verfahren zur Herstellung der entsprechenden aromatischen Polyamingemische, dadurch gekennzeichnet, daß man gemäß den Ansprüchen 1 bis 8 behandelte Polyamingemische verwendet.

10. Verfahren zur Herstellung der kernhydrierten Polyamine oder Vernetzer und Epoxidhärter, dadurch gekennzeichnet, daß man gemäß den Ansprüchen 1 bis 8 behandelte aromatische Polyamingemische verwendet.

11. Verfahren zur Herstellung von Polyurethankunststoffen, dadurch gekennzeichnet, daß gemäß den Ansprüchen 1 bis 8 behandelte aromatische Polyamingemische verwendet.

## Claims

1. Process for the fractionation and purification of aromatic polyamine mixtures of the diphenylmethane series, characterised in that
a) the polyamine starting mixture (A) is distributed in a two-phase system consisting of (i) a hydrophobic solvent phase (B), which consists substantially of hydrophobic solvent, and (ii) an aqueous phase (C) consisting substantially of water, a strong acid and auxiliary amine, which amine is virtually insoluble in water and has a boiling point which under normal pressure is at least 20°C below the boiling point of the lowest-boiling component of the starting mixture and at least 20°C above the boiling point of the solvent, by means of an extraction step (7) operating according to the counter-current principle, with thorough mixing of the phases, by introducing the starting polyamine mixture into the extraction step (7) via the aqueous phase, with the proviso that in this two-phase system the sum of the amine equivalents introduced in the mass flows (A), (B) and (C) always exceeds the quantity of the acid equivalents introduced in mass flow (C); the organic phase (D) leaving this extraction step (7) is separated
b) at least partly via an intermediate extraction step (6) and/or
c) with separation off of a sub-stream before or after the continuous extraction step (6) and return of the separated sub-stream to the extraction step (7) and/or to the extraction step (6) via an extraction step (5) arranged in front,
d) in a multi-step distillation (11.1), (11.2), into a first fraction (E) which consists substantially of hydrophobic solvent and is reused in the extraction step (7), a second fraction (F) consisting substantially of auxiliary amine and a distillation residue (G) consisting substantially of a first polyamine fraction, and
e) the aqueous phase (H) leaving the extraction step (7) is passed into a neutralisation step (8), the acid present in the aqueous phase is neutralised with bases and then, in a phase-separation step, is mechanically separated into an aqueous phase containing the acid in the form of its neutral salts and into an organic phase containing substantially polyamine and
f) the organic phase (J) accumulating in the neutralisation step (8), after passage through a washing step (9), in a distillation step (12) is worked up at least partially into a distillation fraction (K) containing the portions of hydrophobic solvent present in (J) and into a second polyamine fraction accumulating as distillation residue (L).

2. Process according to claim 1, characterised in that
b) the organic phase (D) accumulating in the extraction step (7) is at least partly extracted in counter-current in an intermediate extraction step (6) with at least a part quantity of the aqueous acid (mass flow X) and/or water from mass flow (Y) and/or auxiliary amine, and/or is extracted in counter-current with the entire quantity of the aqueous phase (Q) accumulating in the extraction step (5) arranged in front, the resulting aqueous phase (N) in the intermediate extraction step (6) is passed to the extraction step (7) and the organic phase (O) accumulating in the intermediate extraction step (6) is passed to the working-up step (11).

3. Process according to claims 1 to 2, characterised in that
c) a sub-stream of the organic phase (D) leaving the extraction step (7) and/or
a sub-stream of-the organic phase (O) leaving the intermediate extraction step (6) is/are separated off
and, in an extraction step (5) arranged in front, is extracted in counter-current with a part quantity of the aqueous phase available as mass flow (X),
the organic mass flow (P) used in the extraction step (5) is so calculated that in (5) there is as extensive a transition as possible of the polyamine present in the said organic mass flow (P) into the aqueous phase (Q),
the resulting aqueous phase (Q) in the extraction step (5) arranged in front, after addition of water from mass flow (Y) and/or auxiliary amine, is passed to the extraction step (6) and
the organic phase (R), depleted of polyamine and accumulating in the extraction step (5) arranged in front, is passed to the extraction step (7) and/or to the extraction step (6).

4. Process according to one or more of claims 1 to 3, characterised in that aniline is used as auxiliary amine.

5. Process according to one or more of claims 1 to 3, characterised in that 2,6-dimethylaniline is used as auxiliary amine.

6. Process according to one or more of claims 1 to 3, characterised in that xylidine mixtures are used as auxiliary amine.

7. Process according to one or more of claims 1 to 3, characterised in that 2-methyl-6-ethylaniline is used as auxiliary amine.

8. Process according to one or more of claims 1 to 7, characterised in that the polyamine mixture of the diphenylmethane series used is a polyamine mixture such as is obtained in the acid-catalysed aniline/formaldehyde condensation.

9. Process for the preparation of the corresponding aromatic polyamine mixtures, characterised in that polyamine mixtures treated according to claims 1 to 8 are used.

10. Process for the preparation of ring-hydrogenated polyamines or cross-linking agents and epoxy curing agents, characterised in that aromatic polyamine mixtures treated according to claims 1 to 8 are used.

11. Process for the production of polyurethane plastics, characterised in that aromatic polyamine mixtures treated according to claims 1 to 8 are used.

## Revendications

1. Procédé pour fractionner et purifier des mélanges de polyamines aromatiques de la série du diphénylméthane, caractérisé en ce que
a) on partage le mélange des polyamines de départ (A) dans un système à deux phases consistant en (i) une phase de solvant hydrophobe (B) qui consiste elle-même essentiellement en un solvant hydrophobe et (ii) une phase aqueuse (C) consistant essentiellement en eau, un acide fort et une amine auxiliaire pratiquement insoluble dans l'eau et ayant, à pression normale, un point d'ébullition situé à au moins 20°C au-dessous du-point d'ébullition du composant bouillant le plus bas du mélange initial et à au moins 20°C au-dessus du point d'ébullition du solvant, grâce à un stade d'extraction (7) opérant selon le principe du contre-courant, avec mélange des phases, en introduisant le mélange des polyamines de départ par la phase aqueuse au stade d'extraction (7), sous réserve que dans ce système à deux phases, la somme des équivalents d'amines introduits dans les courants (A), (B) et (C) dépasse en permanence le nombre des équivalents d'acides introduits dans le courant (C),
b) on sépare la phase organique (D) quittant ce stade d'extraction (7), en partie au moins, en passant par un stade d'extraction intermédiaire (6) et/ou
c) en séparant un courant partiel avant ou après le stade d'extraction (6) à traverser et en recyclant le courant partiel séparé au stade d'extraction (7) et/ou au stade d'extraction (6) par l'intermédiaire d'un stade d'extraction préalable (5),
d) dans une distillation à plusieurs étages (11.1), (11.2) en une première fraction (E) réutilisée au stade d'extraction (7), qui consiste essentiellement en le solvant hydrophobe, une deuxième fraction (F) qui consiste essentiellement en l'amine auxiliaire, et un résidu de distillation (G) qui consiste essentiellement en une première fraction de polyamines, et
e) on envoie la phase aqueuse (H) sortant du stade d'extraction (7) à un stade de neutralisation (8) où on neutralise l'acide contenu dans la phase aqueuse puis on la soumet à séparation mécanique dans un stade séparation de phases, en une phase aqueuse contenant l'acide à l'état de sels neutres et une phase organique contenant essentiellement des polyamines,
f) on traite la phase organique (J) quittant le stade de neutralisation (8), après passage dans un stade lavage (9), en partie au moins, dans un étage distillation, où on la sépare en une fraction de distillat (K) contenant les fractions de solvant hydrophobe contenues dans (J), et un résidu de distillation (L) consistant en une deuxième fraction de polyamines.

2. Procédé selon revendication 1, caractérisé en ce que
b) la phase organique (D) sortant du stade d'extraction (7) est extraite à contrecourant, en partie au moins, dans un stade d'extraction intermédiaire (6) par une partie au moins de l'acide aqueux (courant X) et/ou d'eau du courant (Y) et/ou d'amine auxiliaire, et/ou extraite à contre-courant par la totalité de la phase aqueuse (Q) sortant du stade d'extraction préalable (5), la phase aqueuse (N) sortant du stade d'extraction intermédiaire (6) est envoyée au stade d'extraction (7) et la phase organique (O) sortant du stade d'extraction intermédiaire (6) est envoyée au stade isolement (11).

3. Procédé selon les revendication 1 et 2, caractérisé en ce que
c) on sépare un courant partiel de la phase organique (D) quittant le stade d'extraction (7), et/ou
un courant partiel de la phase organique (O) quittant le stade d'extraction intermédiaire (6)
et on l'extrait à contre-courant dans un stade d'extraction préalable (5) par une partie de la phase aqueuse disponible sous forme du courant (X),
on règle le débit du courant organique (P) envoyé au stade d'extraction (5) en sorte que, dans ce stade d'extraction, il se produise un transfert aussi complet que possible des polyamines contenues dans le courant organique (P) vers la phase aqueuse (Q),
on envoie la phase aqueuse (Q) sortant du stade d'extraction préalable (5), après addition d'eau du courant (Y) et/ou d'amine auxiliaire, au stade d'extraction (6) et
on envoie la phase organique (R) appauvrie en polyamines, quittant le stade d'extraction préalable (5) au stade d'extraction (7) et/ou au stade d'extraction(6).

4. Procédé selon une ou plusieurs des revendications 1 à 3, caractérisé en ce que l'amine auxiliaire utilisée est l'aniline.

5. Procédé selon une ou plusieurs des revendications 1 à 3, caractérisé en ce que l'amine auxiliaire utilisée est la 2,6-diméthylaniline.

6. Procédé selon une ou plusieurs des revendications 1 à 3, caractérisé en ce que l'amine auxiliaire utilisée consiste en un mélange de xylidines.

7. Procédé selon une ou plusieurs des revendications 1 à 3, caractérisé en ce que l'amine auxiliaire utilisée est la 2-méthyl-6-éthylaniline.

8. Procédé selon une ou plusieurs des revendications 1 à 7, caractérisé en ce que l'on utilise en tant que mélange de polyamines de la série du diphenylméthane un mélange de polyamines tel qu'obtenu à la condensation aniline/formaldéhyde catalysée par un acide.

9. Procédé de préparation des mélanges de polyamines aromatiques correspondants, caractérisé en ce que l'on utilise des mélanges de polyamines traités selon les revendications 1 à 8.

10. Procédé de préparation de polyamines hydrogénées dans les noyaux ou d'agents réticulants et durcisseurs d'époxyde, caractérisé en ce que l'on utilise les mélanges de polyamines aromatiques traités selon les revendications 1 à 8.

11. Procédé de préparation de résines synthétiques de polyuréthanes, caractérisé en ce que l'on utilise des mélanges de polyamines aromatiques traités selon les revendications 1 à 8.
